# NEW EUROPEAN PATENT SPECIFICATION

(11) **EP 3 886 579 B2**
(45) Date of publication and mention of the opposition decision: **24.12.2025**
(45) Mention of the grant of the patent: 07.06.2023
(21) Application number: 19889740.7
(22) Date of filing: 29.11.2019
(51) Int. Cl.: A01N 1/02

(54) **EX VIVO ORGAN TREATMENT WITH PEG-PHOSPHOLIPID MOLECULES**
EX-VIVO-ORGANBEHANDLUNG MIT PEG-PHOSPHOLIPID-MOLEKÜLEN
TRAITEMENT D'ORGANE EX VIVO AVEC DES MOLÉCULES DE PEG-PHOSPHOLIPIDE

(30) Priority: 30.11.2018 SE 1851492
(43) Date of publication of application: 06.10.2021
(73) Proprietor: iCoat Medical AB, 113 34 Stockholm (SE)
(72) Inventor: NILSSON, Bo, 756 55 Uppsala (SE); NILSSON EKDAHL, Kristina, 756 55 Uppsala (SE); JENSEN WAERN, Marianne, 741 93 Knivsta (SE); TERAMURA, Yuji, Tokyo, Tokyo 112--0011 (JP); BIGLARNIA, Alireza, Malmö 217 75 (SE)
(74) Representative: Barker Brettell Sweden AB
(86) International application number: PCT/SE2019/051215
(87) International publication number: WO 2020/112018

(56) References cited:
- EP-A1- 1 856 973
- WO-A1-99/08514
- WO-A1-99/08514
- WO-A1-99/08514
- US-A1- 2014 007 961
- US-A1- 2014 007 961
- EKDAHL KRISTINA N ET AL: "Protective role of PEG conjugated phospholipid in reducing ischemic reperfusion injury in two allogeneic pig kidney transplant models", IMMUNOBIOLOGY, vol. 221, no. 10, 1 October 2016 (2016-10-01) - 1 October 2016 (2016-10-01), pages 1184, XP029684449, ISSN: 0171-2985, DOI: 10.1016/J.IMBIO.2016.06.133
- NILSSON PER H., EKDAHL KRISTINA N., MAGNUSSON PEETRA U., QU HONGCHANG, IWATA HIROO, RICKLIN DANIEL, HONG JAAN, LAMBRIS JOHN D., NI: "Autoregulation of thromboinflammation on biomaterial surfaces by a multicomponent therapeutic coating", BIOMATERIALS, ELSEVIER, AMSTERDAM, NL, vol. 34, no. 4, 1 January 2013 (2013-01-01), AMSTERDAM, NL , pages 985 - 994, XP093143412, ISSN: 0142-9612, DOI: 10.1016/j.biomaterials.2012.10.040
- KRISTINA N. EKDAHL; YUJI TERAMURA; OSAMA A. HAMAD; SANA ASIF; CLAUDIA DUEHRKOP; KARIN FROMELL; ELISABET GUSTAFSON; JAAN HONG; HUDA: "Dangerous liaisons: complement, coagulation, and kallikrein/kinin cross‐talk act as a linchpin in the events leading to thromboinflammation", IMMUNOLOGICAL REVIEWS, WILEY-BLACKWELL PUBLISHING, INC., US, vol. 274, no. 1, 26 October 2016 (2016-10-26), US , pages 245 - 269, XP071456188, ISSN: 0105-2896, DOI: 10.1111/imr.12471
- AGGARWAL, S. ; KANG, Y. ; FREEMAN, J.A. ; FORTUNATO, F.L. ; PINSKY, M.R.: "Postreperfusion syndrome: Hypotension after reperfusion of the transplanted liver", JOURNAL OF CRITICAL CARE, GRUNE AND STRATTON, ORLANDO, FL, US, vol. 8, no. 3, 1 September 1993 (1993-09-01), US , pages 154 - 160, XP023060532, ISSN: 0883-9441, DOI: 10.1016/0883-9441(93)90021-C
- BEJAOUI MOHAMED, PANTAZI EIRINI, FOLCH-PUY EMMA, PANISELLO ARNAU, CALVO MARÍA, PASUT GIANFRANCO, RIMOLA ANTONI, NAVASA MIQUEL, ADA: "Protective Effect of Intravenous High Molecular Weight Polyethylene Glycol on Fatty Liver Preservation", BIOMED RESEARCH INTERNATIONAL, HINDAWI PUBLISHING CORPORATION, vol. 2015, 1 January 2015 (2015-01-01), pages 1 - 10, XP093143423, ISSN: 2314-6133, DOI: 10.1155/2015/794287
- MOHAMED BEJAOUI, EIRINI PANTAZI, MARIA CALVO, EMMA FOLCH-PUY, ANNA SERAFíN, GIANFRANCO PASUT, ARNAU PANISELLO, RENé ADAM: "Polyethylene Glycol Preconditioning: An Effective Strategy to Prevent Liver Ischemia Reperfusion Injury", OXIDATIVE MEDICINE AND CELLULAR LONGEVITY, HINDAWI PUBLISHING CORPORATION, US, vol. 2016, 1 January 2016 (2016-01-01), US , pages 1 - 10, XP055674073, ISSN: 1942-0900, DOI: 10.1155/2016/9096549
- TAKAHAMA HIROYUKI; MINAMINO TETSUO; ASANUMA HIROSHI; FUJITA MASASHI; WAKENO MASAKATSU; ASAKURA MASANORI; KIM JHYOONG; TAKASHIMA SE: "Polyethylene-glycol coated liposomal adenosine attenuates hypotension and bradycardia and augments cardioprotection against ischemia/repertusion injury in rats - Nano-size drug delivery system targeted to infarcted myocardium", CIRCULATION, AMERICAN HEART ASSOCIATION, US, vol. 116, no. 16, Suppl. S, 16 October 2007 (2007-10-16) - 7 November 2007 (2007-11-07), US , pages 97, XP008136987, ISSN: 0009-7322
- ABOLFAZL AKBARZADEH, REZAEI-SADABADY ROGAIE, DAVARAN SOODABEH, JOO SANG WOO, ZARGHAMI NOSRATOLLAH, HANIFEHPOUR YOUNES, SAMIEI MOHA: "Liposome: classification, preparation, and applications", 1 January 2013 (2013-01-01), XP055178982, Retrieved from the Internet <URL:http://www.nanoscalereslett.com/content/pdf/1556-276X-8-102.pdf> [retrieved on 20150324], DOI: 10.1186/1556-276X-8-102
- PASUT GIANFRANCO, PANISELLO ARNAU, FOLCH-PUY EMMA, LOPEZ ALEXANDRE, CASTRO-BENÍTEZ CARLOS, CALVO MARIA, CARBONELL TERESA, GARCÍA-G: "Polyethylene glycols: An effective strategy for limiting liver ischemia reperfusion injury", WORLD JOURNAL OF GASTROENTEROLOGY, WJG PRESS, CN, vol. 22, no. 28, 1 January 2016 (2016-01-01), CN , pages 6501, XP093143435, ISSN: 1007-9327, DOI: 10.3748/wjg.v22.i28.6501
- GUSTAFSON ELISABET: "Thromboinflammation in a Model of Hepatocyte Transplantation", DOCTORAL DISSERTATION, UPPSALA UNIVERSITY, UPPSALA UNIVERSITET, INSTITUTIONEN FÖR KVINNORS OCH BARNS HÄLSA, 10 June 2016 (2016-06-10), XP093143443, Retrieved from the Internet <URL:https://www.diva-portal.org/smash/get/diva2:922111/FULLTEXT01.pdf> [retrieved on 20240320]
- SONG D., OLANO M., WILSON D. F., PASTUSZKO A., TAMMELA O., NHO K., SHORR R.G.L.: "Comparison of the efficacy of blood and polyethylene glycol‐hemoglobin in recovery of newborn piglets from hemorrhagic hypotension: effect on blood pressure, cortical oxygen, and extracellular dopamine in the brain", TRANSFUSION, AMERICAN ASSOCIATION OF BLOOD BANKS, BETHESDA, MD., US, vol. 35, no. 7, 1 July 1995 (1995-07-01), US , pages 552 - 558, XP093143583, ISSN: 0041-1132, DOI: 10.1046/j.1537-2995.1995.35795357876.x
- JACKSON SHAUN P., DARBOUSSET ROXANE, SCHOENWAELDER SIMONE M.: "Thromboinflammation: challenges of therapeutically targeting coagulation and other host defense mechanisms", BLOOD, AMERICAN SOCIETY OF HEMATOLOGY, US, vol. 133, no. 9, 28 February 2019 (2019-02-28), US , pages 906 - 918, XP055976337, ISSN: 0006-4971, DOI: 10.1182/blood-2018-11-882993
- LISSONI P., PITTALIS S., ARDIZZOIA A., BRIVIO F., BARNI S., TANCINI G., PELIZZONI F., MAESTRONI G. J. M., ZUBELEWICZ B., BRACZKOWS: "Prevention of cytokine-induced hypotension in cancer patients by the pineal hormone melatonin", SUPPORTIVE CARE IN CANCER, SPRINGER BERLIN HEIDELBERG, BERLIN/HEIDELBERG, vol. 4, no. 4, 1 July 1996 (1996-07-01), Berlin/Heidelberg, pages 313 - 316, XP093143587, ISSN: 0941-4355, DOI: 10.1007/BF01358887
- "Critical care medicine : principles of diagnosis and management in the adult", 1 January 2008, ELSEVIER SAUNDERS, Philadelphia, ISBN: 978-0-323-04841-5, article STEPHEN TRZECIAK, R. PHILLIP DELLINGER, JOSEPH E. PARRILLO: "Chapter 24: Septic Shock", pages: 439 - 452, XP009553476, DOI: 10.1016/B978-032304841-5.50026-1
- MAL HERVÉ, DEHOUX MONIQUE, SLEIMAN CHARLES, BOCZKOWSKI JORGE, LESÈCHE GUY, PARIENTE RENÉ, FOURNIER MICHEL: "Early Release of Proinflammatory Cytokines After Lung Transplantation", CHEST, ELSEVIER, NORTHBROOK, IL, vol. 113, no. 3, 1 March 1998 (1998-03-01), Northbrook, IL, pages 645 - 651, XP009553272, ISSN: 0012-3692, DOI: 10.1378/chest.113.3.645
- ARRANZ J, SORIANO A, GARCIA I, GARCÍA I, CONCEPCIÓN M.T, NAVARRO J, ARTEAGA A, FILELLA X, BRAVO P, BARRERA M, ESCRIBANO S, JIMÉNEZ: "Effect of proinflammatory cytokines (IL-6, TNF-α, IL-1β) on hemodynamic performance during orthotopic liver transplantation", TRANSPLANTATION PROCEEDINGS, ELSEVIER INC., ORLANDO, FL; US, vol. 35, no. 5, 1 August 2003 (2003-08-01), ORLANDO, FL; US , pages 1884 - 1887, XP093143593, ISSN: 0041-1345, DOI: 10.1016/S0041-1345(03)00603-1
- HASHIMOTO NAOKI, TAKEYOSHI IZUMI, TSUTSUMI HIROFUMI, SUNOSE YUTAKA, TOKUMINE MASAHIKO, TOTSUKA OSAMU, OHWADA SUSUMU, YOKOE TAKAO, : "Effects of a bradykinin B2 receptor antagonist, FR173657, on pulmonary ischemia–reperfusion injury in dogs", JOURNAL OF HEART AND LUNG TRANSPLANTATION, ELSEVIER, AMSTERDAM, NL, vol. 21, no. 9, 1 September 2002 (2002-09-01), AMSTERDAM, NL , pages 1022 - 1029, XP093143597, ISSN: 1053-2498, DOI: 10.1016/S1053-2498(02)00405-9
- ESSANDOH MICHAEL, OTEY ANDREW JOSEPH, DALIA ADAM, DEWHIRST ELISABETH, SPRINGER ANDREW, HENRY MITCHELL: "Refractory Hypotension after Liver Allograft Reperfusion: A Case of Dynamic Left Ventricular Outflow Tract Obstruction", FRONTIERS IN MEDICINE, FRONTIERS MEDIA, FR, vol. 3, FR , XP093143599, ISSN: 2296-858X, DOI: 10.3389/fmed.2016.00003
- ANTONIO SINISCALCHI, LORENZO GAMBERINI, CRISTIANA LAICI, TOMMASO BARDI, LAURA LORENZINI, STEFANO FAENZA, GIORGIO ERCOLANI, SINISCA: "Post reperfusion syndrome during liver transplantation: From pathophysiology to therapy and preventive strategies", WORLD JOURNAL OF GASTROENTEROLOGY, WJG PRESS, CN, vol. 22, no. 4, 1 January 2016 (2016-01-01), CN , pages 1551, XP055714649, ISSN: 1007-9327, DOI: 10.3748/wjg.v22.i4.1551
- KIM YOUNG-KUG, LEE KICHANG, HWANG GYU-SAM, COHEN RICHARD J.: "Sympathetic withdrawal is associated with hypotension after hepatic reperfusion", CLINICAL AUTONOMIC RESEARCH, RAPID COMMUNICATIONS, OXFORD,, GB, vol. 23, no. 3, 1 June 2013 (2013-06-01), GB , pages 123 - 131, XP093143601, ISSN: 0959-9851, DOI: 10.1007/s10286-013-0191-0
- Francesco Maione, et al.”Long-Lasting Anti-Inflammantory and Antinociceptive Effects of Acute Ammonium Glycyrrhizinate…”
- Steven R. Bruhl et al. Post-reperfusion Syndrome during Renal Transplantation : A Retrospective Study
- EKDAHL, KRISTINA N ET AL.: "Protective role of PEG conjugated phospholipid in reducing ischemic reperfusion injury in to allogeneic pig kidney transplant models", ABSTRACT/LMMUNOBIOLOGY, vol. 221, 2016, pages 1131 - 1225, XP029684449
- ASIF, SANA ET AL.: "Conjugation of human recombinant SD39 to primary human hepatocytes protects against thromboinflammation", XENOTRANSPLANTATION, vol. 22, no. 1, November 2015 (2015-11-01), Melbourne, VIC, Australia, pages S87, XP55714648
- SINISCALCHI, ANTONIO ET AL.: "Post reperfusion syndrome during liver transplantation: From pathophysiology to therapy and preventive strategies", WORLD J GAASTROENTEROL, vol. 22, no. 4, 2016, pages 1551 - 1569, XP055714649

## Description

### TECHNICAL FIELD

The invention generally relates to *ex vivo* organ treatments, and in particular to such *ex vivo* organ treatments capable of inhibiting or preventing thromboinflammation and hypotension induced by organ reperfusion.

### BACKGROUND

Ischemia reperfusion (I/R) injury (IRI) is a major contributor to the pathology of several conditions including transplantation, thrombotic disorders, sepsis and cardiopulmonary bypass, which all induces thromboinflammation.

To date kidney transplantation is the most effective treatment for patients with end-stage renal disease. Despite impressive improvements in treatment results compared to the introduction in the 1960s, there is still a graft loss of 25 % after 5 years and almost 50 % after 10 years. The main target for improving kidney transplantation results is IRI since IRI evokes acute renal failure, delayed graft function and remote organ failure. Immunosuppression does not affect the humoral innate immune system to a great extent and therefore does not attenuate IRI. The transplantation field is also hampered by organ shortage. One option for increasing the donor pool is an increased use of marginal organs, such as those from deceased-after-cardiac-death (DCD) donors. However, these organs are also severely affected by IRI.

During ischemia the metabolism switches into an anaerobic condition that ultimately results in an inflammatory state, which is triggered by up- and down-regulation of a number of genes including nuclear factor kappa-light-chain-enhancer of activated B cells (NF-κB) and thereby cytokines, chemokines and tissue factor (TF), but also genes that protect against ischemia, such as heme oxygenase 1 (HO-1), hypoxia-inducible factors (HIF) 1 and 2 and nitrotyrosine. Under normoxic conditions, the endothelial cell surface is covered by a negatively charged "hairy" mesh called glycocalyx (GCX), which comprises a complex network of proteoglycans, glycosaminoglycans and glycoproteins that incorporates anti-inflammatory and anti-coagulant proteins to ensure the microvascular homeostatsis and prevent thromboinflammation. Moreover, the thick GCX shields the cell surface and adhesion molecules, such as intercellular adhesion molecule 1 (ICAM-1), vascular cell adhesion molecule 1 (VCAM-1) and E-selectin, thereby preventing recruitment of leukocytes and platelets onto the endothelial surface. The GCX is very sensitive to oxidative stress and ischemia-induced structural damage, which often results in a rapid loss of the GCX coat. This is caused by the expression of heparinase and metalloproteinases in the endothelial cell lining, which results in cleavage and break down of the GCX that protects the endothelial cells from innate immune attack. The GCX harbors a number of growth factors, such as hepatocyte growth factor (HGF) and vascular endothelial growth factor (VEGF), which thereby are released during GCX break down. This will also result in that regulators of the complement, coagulation and the contact systems will be released from the cell surface. Such regulators are, for instance, antithrombin (AT), protein C, tissue factor pathway inhibitor (TFPI), C4b-binding protein (C4BP), Factor H and C1-inhibitor (C1-INH) with the consequence that the endothelial cell surface will be unprotected against attacks by the complement, coagulation and contact systems. Finally, if the ischemia lingers, the adenosine triphosphate (ATP) stores are depleted, leading to apoptosis and necrosis.

Reperfusion of the ischemic organ induces a repair process, but initially it starts a devastating injury to the organ due to the pathological changes already induced by the ischemia. The rapid oxygenation generates injury involving the generation of reactive oxygen species (ROS) and the unprotected endothelial surface triggers a destructive activation of the complement and the contact systems. In transplantation, collectin-11/12 (CL-1 1/12) expression in the kidney graft recognizes the ischemic cells and contributes to the complement activation via the lectin pathway (LP). Also other recognition molecules, such as mannose-binding lectin (MBL) and Ficolin-2, of the LP have been linked to complement activation. Evidence of complement activation is deposition of C4d on the endothelial cells combined with membrane attack complex (MAC) deposition in the near-vessel parenchyma, indicating cell damage in these areas. Triggered by the up-regulated TF expression and the contact system activation, fibrin deposition reveals that the coagulation system is engaged. In combination with complement activation, up-regulation and exposure of von Willebrand factor (vWF) followed by binding and activation of platelets and polymorphonuclear neutrophils (PMNs) to the endothelial surface, thromboinflammation is elicited. Much of this thromboinflammation is driven by locally produced complement factors clearly demonstrated in a mouse model of kidney transplantation, where the knockout of C3 protects the transplanted organ from rejection.

Reperfusion of the transplanted organ is also associated with hemodynamic instability post-reperfusion. This phenomenon, which previously has been described as post reperfusion syndrome (PRS), was first described in liver transplant recipients who experienced severe hemodynamic instability after reperfusion of the liver graft. However, this phenomenon is also observed in kidney and pancreas transplant recipients, who often suffers from instant hemodynamic instability after reperfusion. Consequently, the rapid decrease of systemic arterial pressure results in impaired graft perfusion and prolongation of the warm ischemia of the graft. These patients can suffer from delayed graft function, increased risk for rejection and premature graft loss.

The amphiphilic polymer polyethylene glycol)-phospholipid (PEG-conjugated phospholipid or simply PEG-phospholipid) is spontaneously incorporated into the lipid bilayer membrane of cell membranes by hydrophobic interactions [1, 2]. Furthermore, the creation of an artificial cell surface layer by PEG-phospholipid could attenuate instant blood mediated inflammatory reaction (IBMIR) and prolong both the survival and the function of islets after intra-portal infusion in diabetic mice [3, 6].

It has previously been shown that PEG-phospholipid conjugated with innate immune regulators, such as apyrase and Factor H, protects cells and material surfaces against innate immune attack *in vitro* [4, 5].

Ekdahl et al., Protective role of PEG conjugated phospholipid in reducing ischemic reperfusion injury in two allogenic pig kidney transplant models, Immunology 221(10): 1184, 2016, discloses that PEG-lipid coatings is very successful in lowering the innate immune response during allogeneic transplantation in large animal models.

There is, however, a need for improvements in connection with organ transplantation.

### SUMMARY

It is a general objective to provide improvements in connection with organ transplantation.

This and other objectives are met by embodiments as disclosed herein.

The invention is defined in the independent claim. Further embodiments of the invention are defined in the dependent claims.

An *ex vivo* method of treating an organ or a part of the organ comprises *ex vivo* infusing a solution comprising PEG-phospholipid molecules into a vascular system of the organ or the part of the organ. The method also comprises *ex vivo* incubating the solution comprising PEG-phospholipid molecules in the vascular system to enable coating of at least a portion of the endothelial lining of the vascular system with the PEG-phospholipid molecules while keeping the organ or the part of the organ submerged in an organ preservation solution comprising PEG-phospholipid molecules.

An organ graft *ex vivo* treated with PEG-phospholipid molecules had significantly less thromboinflammation as compared to untreated control organ grafts as reflected by the plasma levels of coagulation markers, such as TAT, and complement markers, such as C3a and sC5b-9. Also cytokine expression, particularly IL-6 and IL-1β, was much suppressed in the PEG-phospholipid treated organ grafts. Organ function was improved in the PEG-phospholipid treated organ grafts. PEG-phospholipid treated organ grafts also showed reduced deposition of complement markers, such as C3b and MAC, and expression of C5aR. A highly surprising, but medically significant, effect of *ex vivo* treating an organ graft with PEG-phospholipid molecules was that such PEG-phospholipid treatment was capable of reducing the devastating blood pressure drop in the recipient that is frequently associated with reperfusion of the organ graft. Furthermore, after 4 days PEG-phospholipid treatment was capable of reducing the increase in the T cell-dependent cytokines IL-4 and IL-12, which are associated with activation of the adaptive immune response.

### BRIEF DESCRIPTION OF THE DRAWINGS

The embodiments, together with further objects and advantages thereof, may best be understood by making reference to the following description taken together with the accompanying drawings, in which:

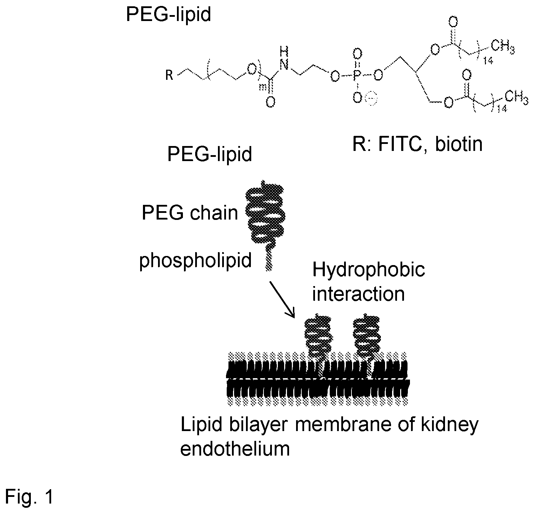

Fig. 1 schematically illustrates the mechanism of hydrophobic interaction of the PEG-lipid molecule to the lipid bilayers of cell membranes.

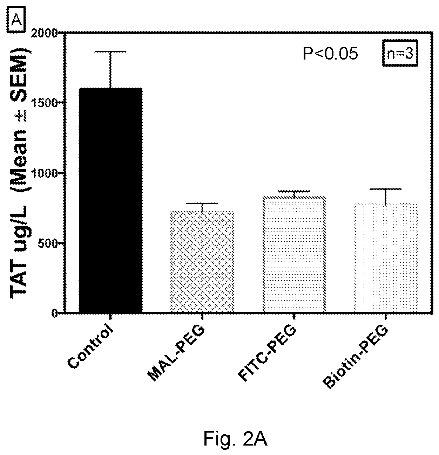

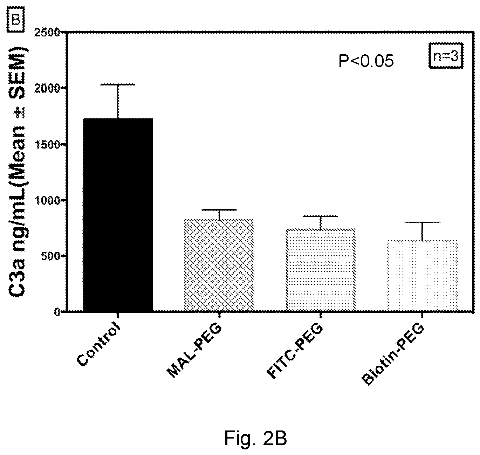

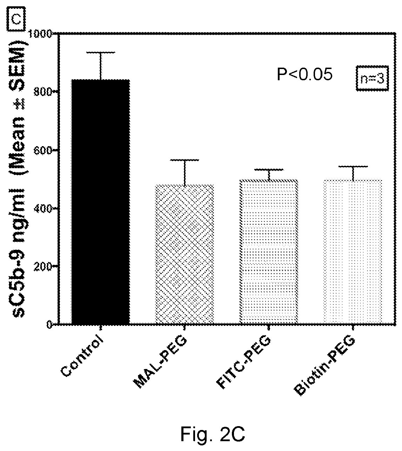

Figs. 2A to 2C illustrate the effect of various functional groups of PEG-phospholipid on coagulation (Fig. 2A) and complement (Figs. 2B and 2C) when coated MSCs were analyzed in an *in vitro* Chandler loop model in comparison to control (uncoated cells).

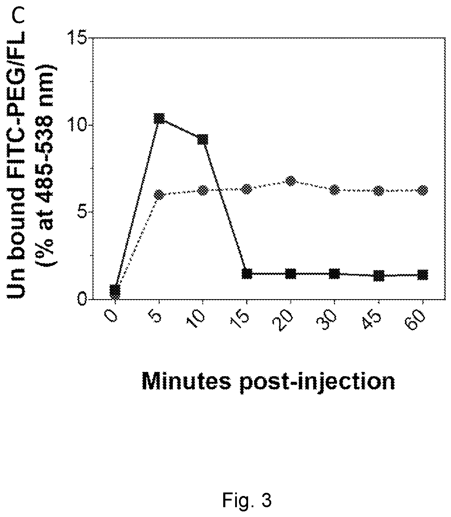

Fig. 3 illustrates fluid-phase concentration during 60 min during perfusion with 25 mL of 2 mg/mL FITC-PEG-phospholipid. Circles indicate without connected kidney and squares indicate with connected kidney. The calculated uptake of PEG-phospholipid was 76 %.

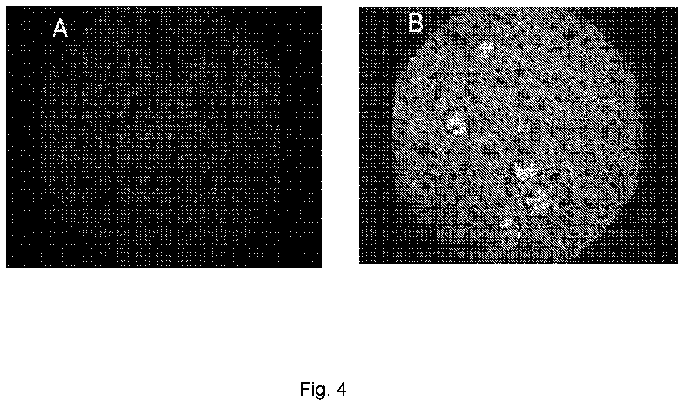

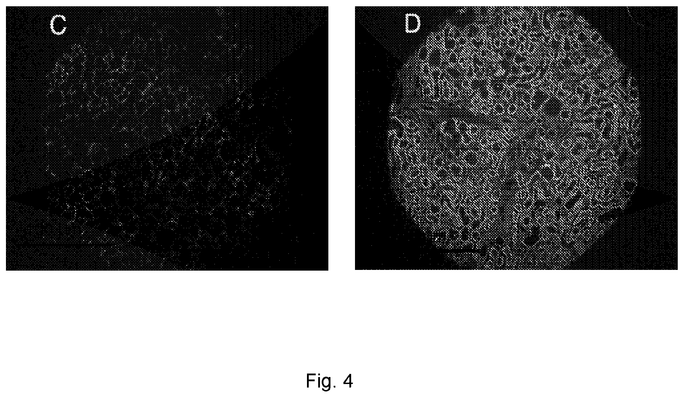

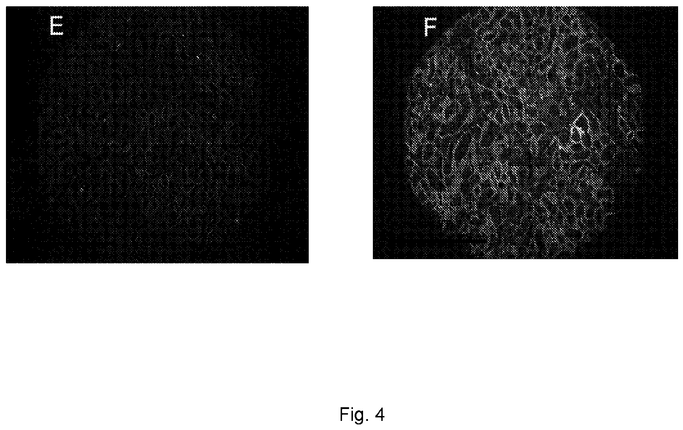

Figs. 4A to 4F illustrate biopsies retrieved from control and treated kidneys at time point 5 min (Fig. 4A: control, Fig. 4B: treated), 60 min (Fig. 4C: control, Fig. 4D: treated) and 360 min (Fig. 4E: control, Fig. 4F: treated) post reperfusion. Kidneys were perfused with biotin-PEG-phospholipid solution and the validation of coating of kidney endothelium with PEG-phospholipid was carried out by counterstaining with streptavidin-488. Sections were analyzed by confocal microscopy.

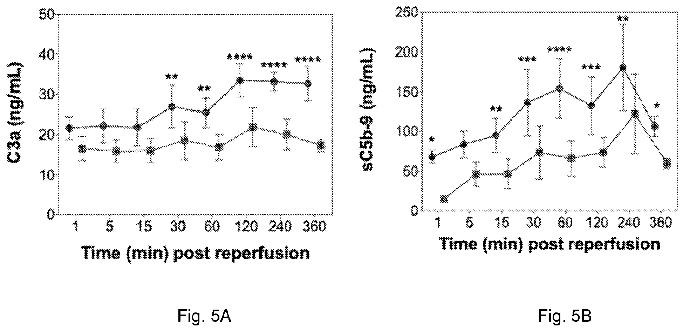

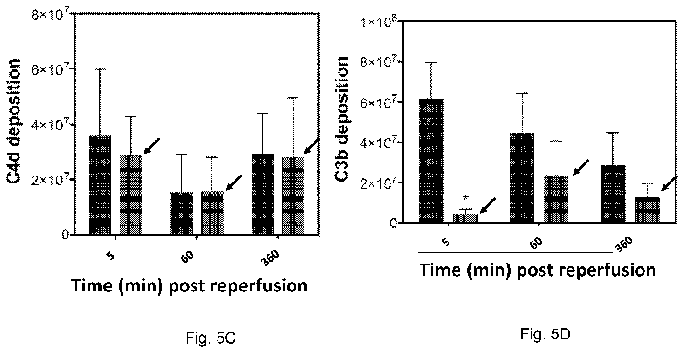

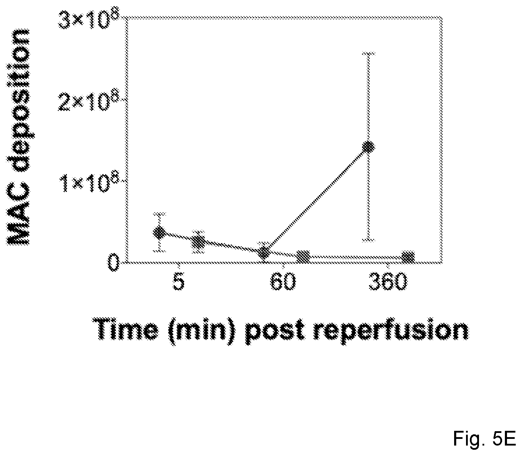

Figs. 5A to 5E illustrate complement activation in the short-term allogeneic porcine kidney transplantation model. Fig. 5A plasma C3a, Fig. 5B sC5b-9, Fig. 5C C4d, Fig. 5D C3b and Fig. 5E MAC deposition in biopsies from the kidney. Squares and bars with arrows represent the PEG-phospholipid treated kidneys and circles and bars without arrows represent the untreated control. * p<0.05; ** p<0.01; *** p<0.001; **** p<0.0001.

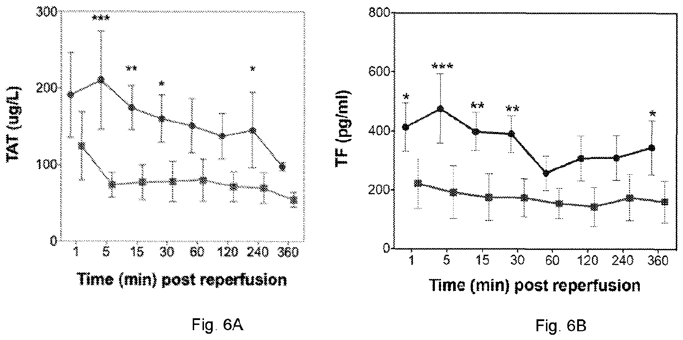

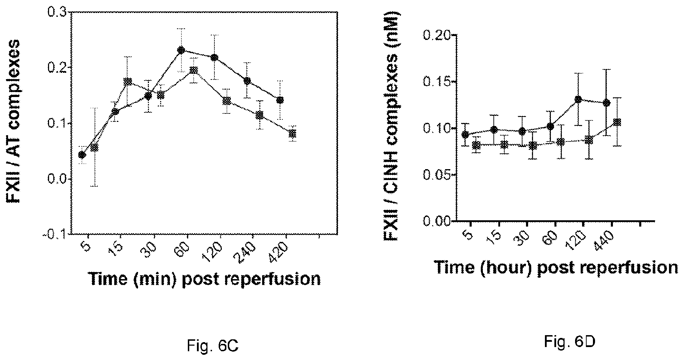

Figs. 6A to 6D illustrate coagulation activation in the short-term allogeneic kidney transplantation model. Fig. 6A plasma TAT, Fig. 6B TF, Fig. 6C FXII/AT and Fig. 6D FXII/C1 INH. Squares represent the PEG-phospholipid treated kidneys and circles represent the untreated control. * p<0.05; ** p<0.01; *** p<0.001.

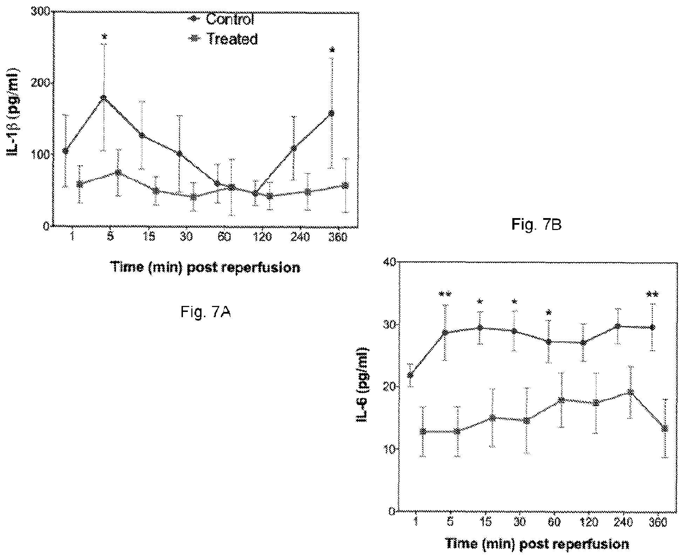

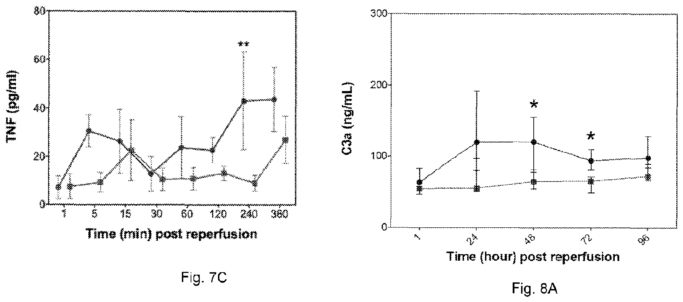

Figs. 7A to 7C illustrate the concentration of proinflammatory cytokines in the short-term allogeneic kidney transplantation model. Fig. 7A plasma IL-1β, Fig. 7B IL-6, and Fig. 7C TNF. Squares represent the PEG-phospholipid treated kidneys and circles represent the untreated control. * p<0.05; ** p<0.01.

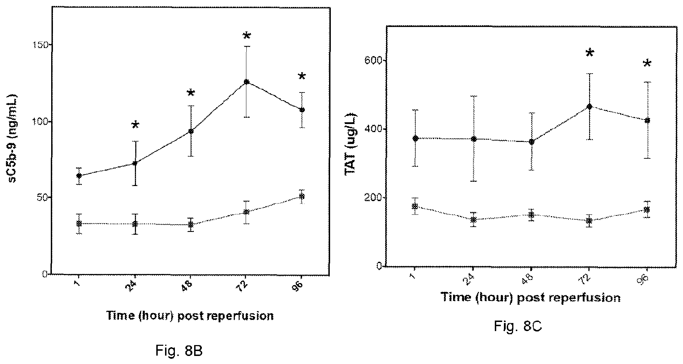

Figs. 8A to 8C illustrate complement and coagulation activation in the long-term allogeneic kidney transplantation model. Fig. 8A plasma C3a, Fig. 8B sC5b-9, and Fig. 8C TAT. Squares represent the PEG-phospholipid treated kidneys and circles represent the untreated control. * p<0.05; ** p<0.01.

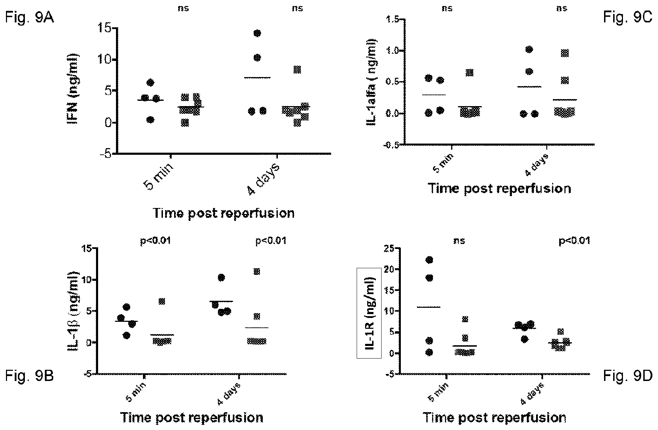

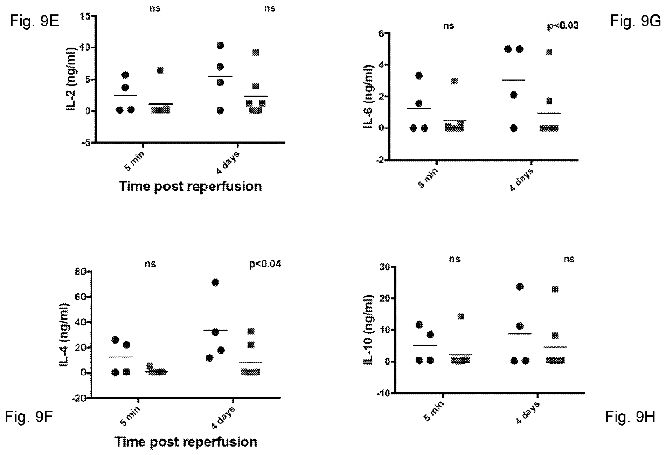

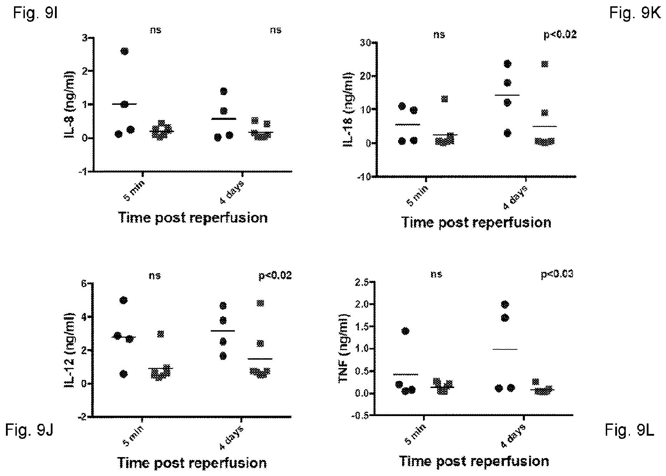

Figs. 9A to 9L illustrate the concentration of cytokines/chemokines in the long-term allogeneic kidney transplantation model. Fig. 9A plasma INFy, Fig. 9B IL-1β, Fig. 9C IL-1α, Fig. 9D IL-1R, Fig. 9E IL-2, Fig. 9F IL-4, Fig. 9G IL-6, Fig. 9H, IL-10, Fig. 9I IL-8, Fig. 9J IL-12, Fig. 9K IL-18 and Fig. 9L TNF. Squares represent the PEG-phospholipid treated kidneys and circles represent the untreated control.

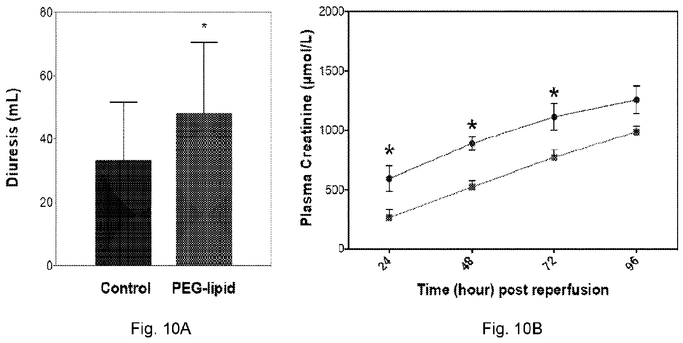

Figs. 10A and 10B illustrate functional evaluation of the PEG-phospholipid treated kidney in the short-term (Fig. 10A) and in the long-term (Fig. 10B) allogeneic kidney transplantation models. In Fig. 10A, diuresis was assessed and, in Fig. 10B, creatinine levels were measured. Squares represent the PEG-phospholipid treated kidneys and circles represent the untreated control. * p<0.05.

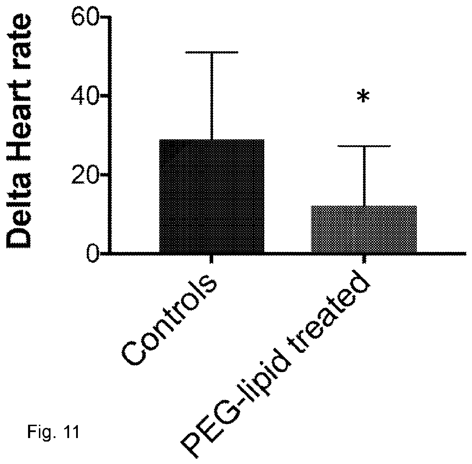

Fig. 11 illustrates functional evaluation with regard to the compensatory increase in pulse rate immediately after reperfusion in the pigs transplanted with the PEG-phospholipid treated kidneys compared to the animals transplanted with the control kidneys in the long-term allogeneic kidney transplantation models. * p<0.05.

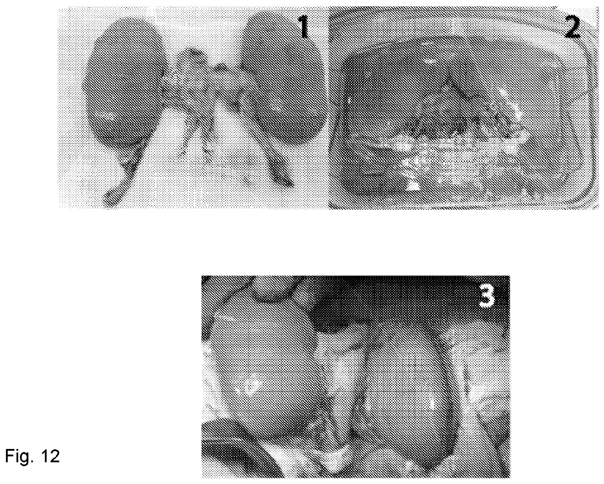

Fig. 12 illustrates gross morphology of porcine kidneys post (1) procurement, (2) static hypothermic perfusion and (3) PEG-phospholipid perfusion.

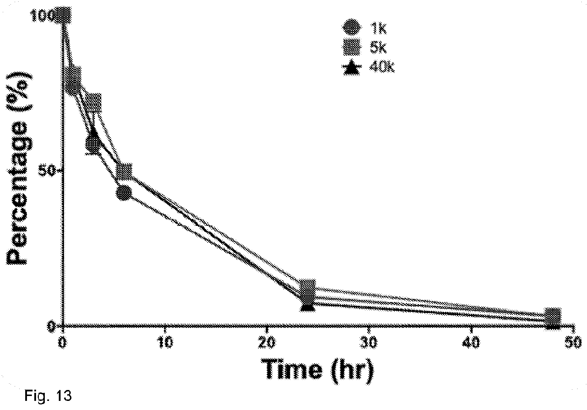

Fig. 13 illustrates retention of PEG-phospholipid molecules with different lengths of the PEG subunit (1 kDa circles, 5 kDa squares and 40 kDa triangles) using CCRF-CEM cells.

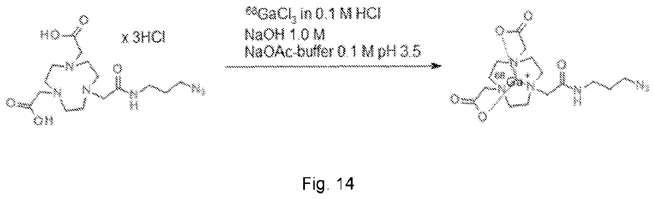

Fig. 14 illustrates synthesis of [⁶⁸Ga]NO2A-propyl-azide.

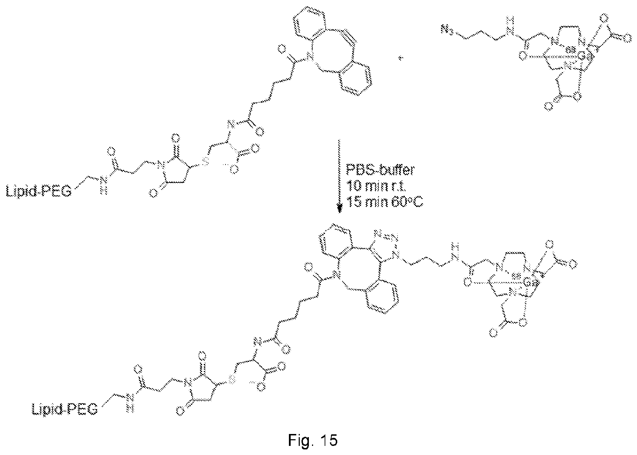

Fig. 15 illustrates synthesis of ⁶⁸Ga-NO2A-PEG-Lipid.

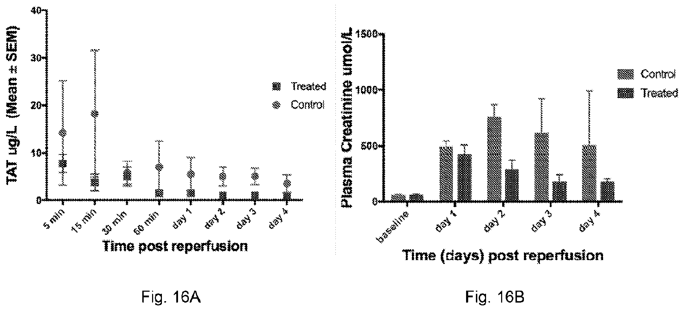

Figs. 16A and 16B illustrate TAT values and plasma creatinine values after incubating pig kidneys for 24 hours in PEG-phospholipid supplemented HTK solution (treated) or only HTK solution (control) at 4°C.

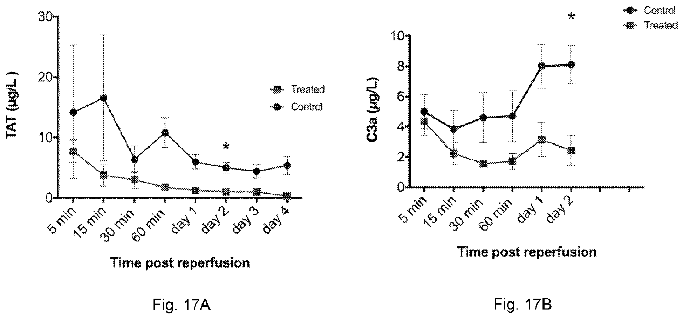

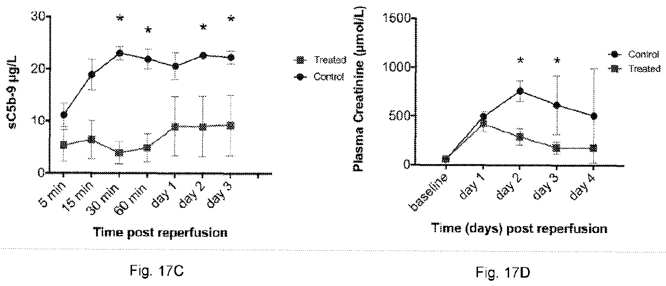

Figs. 17A to 17D illustrate complement and coagulation activation, and creatinine levels in the long-term allogeneic kidney transplantation model, plasma TAT (Fig. 17A), C3a (Fig. 17B), sC5b-9 (Fig. 17C) and creatinine (Fig. 17D). Lines with squares represent the PEG-phospholipid treated kidneys and lines with circles represent the untreated control. * p<0.05.

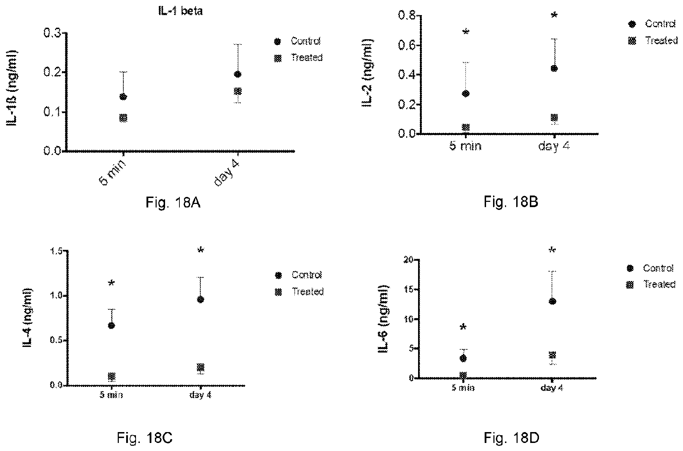

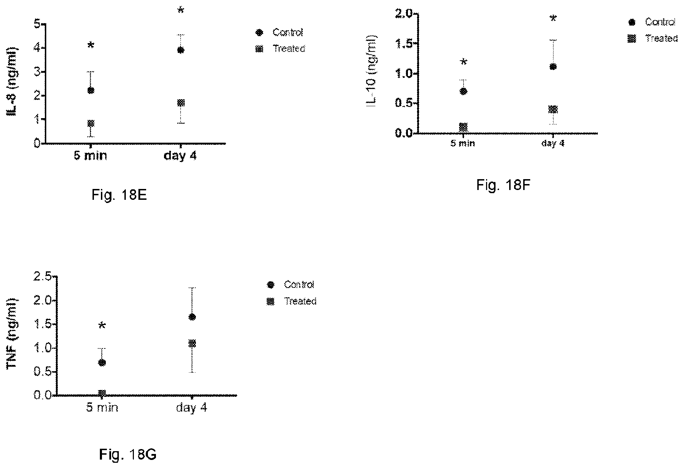

Figs. 18A to 18G illustrate the concentration of cytokines/chemokines in the long-term allogeneic kidney transplantation model, plasma IL-1β (Fig. 18A), IL-2 (Fig. 18B), IL-4 (Fig. 18C), IL-6 (Fig. 18D), IL-8 (Fig. 18E), IL-10 (Fig. 18F) and TNF (Fig. 18G). Squares represent the PEG-phospholipid treated kidneys and circles represent the untreated control. * p<0.05.

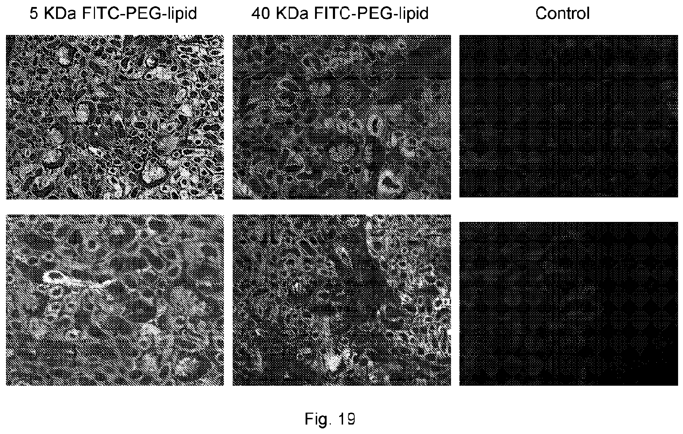

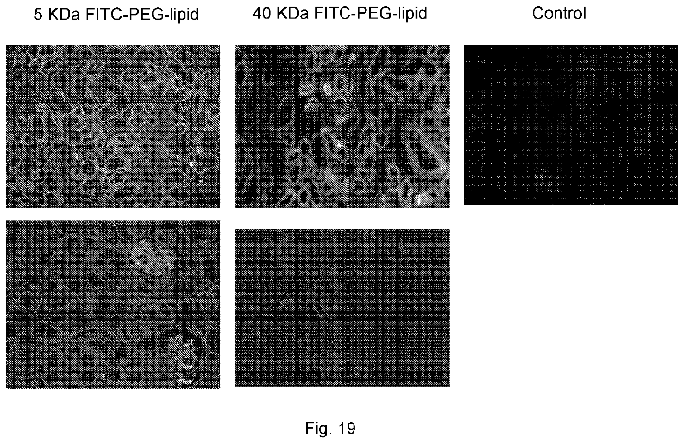

Fig. 19 illustrates the uptake of 5 kDa PEG-lipid and 40 kDa PEG-lipid after infusion of the PEG-lipid molecules into the kidneys from four different pigs with one kidney used for 5 kDa PEG-lipid and the other for 40 kDa PEG-lipid. Kidneys were perfused with FITC-PEG-phospholipid solution and the validation of coating of kidney endothelium and parenchyma with PEG-phospholipid was visualized. Sections were analyzed by confocal microscopy.

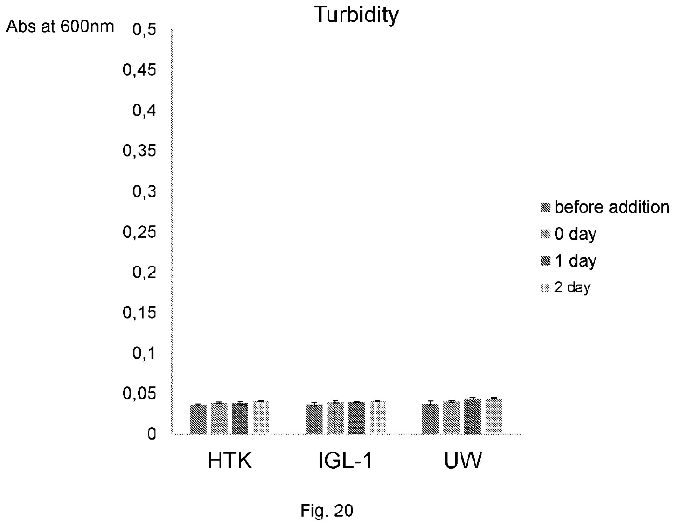

Fig. 20 illustrates the mixability of PEG-lipid molecules in three commonly used perfusion liquids (HTK, IGL-1 and UW). No precipitation was observed in any of these mixtures at a concentration of 2 mg/mL of PEG-lipid.

### DETAILED DESCRIPTION

The invention generally relates to *ex vivo* organ treatments, and in particular to such *ex vivo* organ treatments capable of treating, inhibiting or preventing thromboinflammation, which precedes activation of the adaptive immune system, and hypotension induced by organ reperfusion.

The *ex vivo* method of the invention leads to a significant improvement in organ transplantations and organ treatments by providing a protective shielding or coating of the endothelial lining or endothelium of the vascular system and of the parenchyma of such organs or parts of such organs. This protective coating constitutes an effective protection against thromboinflammation and deleterious effects of ischemia reperfusion (I/R) injury (IRI).

The glycocalyx is essential for ensuring microvascular hemostasis and prevention of thromboinflammation. This glycocalyx is, however, very sensitive to oxidative stress and ischemia-induced damage. Hence, the protective glycocalyx coating is rapidly lost during ischemia that occurs in connection with organ procurement and organ preservation *in vitro.* Once the organ graft is transplanted and reperfused, a repair process is induced, which starts a devastating injury to the organ graft due to the absence or damages to the glycocalyx. Hence, the complement and contact system are activated. Platelets are consumed and leukocytes become activated and infiltrate the organ graft. As a consequence of activation of these systems, cytokines are generated ultimately leading to cell death and graft loss or delayed graft function. This devastating injury to the organ graft is a thromboinflammation, which is primarily triggered by the humoral innate immune system that mainly consists of the cascade systems of the blood, i.e., the complement, contact/coagulation and fibrinolytic systems. Activation of these systems subsequently induces activation of endothelial cells, leukocytes and platelets, finally resulting in thrombotic and inflammatory reactions.

As is shown herein, by *ex vivo* treating an organ or a part of the organ with polyethylene glycol)-phospholipid (PEG-phospholipid) molecules the organ or the part of the organ can be protected against the thromboinflammation. Hence, the PEG-phopshoplipid molecules are capable of binding to the cell membranes of the endothelial lining of the vascular system of the organ or the part of the organ and thereby compensate for the loss of the glycocalyx. Hence, the PEG-phospholipid molecules form a protective coating of the endothelium that inhibits or at least significantly reduces the deleterious effects of thromboinflammation, including lower levels of coagulation and complement activation, suppression of cytokine expression and general improvement of the graft function.

In particular, *ex vivo* treating the organ with PEG-phospholipid molecules led to a significant reduction in cytokines, such as in T-cell specific cytokines including interleukin 4 (IL-4) and IL-12. In non-treated control organs, there was a high level of such cytokines several days following transplantation, which has a negative effect on the continuing immunization and future immunosuppression. Hence, the coating of the endothelium by PEG-phospholipid molecules inhibits the cytokine-induced effects and damages to the organ or the part of the organ after transplantation and also improves the outcome of the immunosuppression after the transplantation.

Another highly surprising effect achieved by *ex vivo* treating organs or part of organs with PEG-phospholipid molecules was the treatment, inhibition or prevention of the hemodynamic instability with increased heart rate and hypotension associated with reperfusion developing after reperfusion of the organ graft. Such a hypotension in connection with the transplantation can result in poor perfusion, organ failure and even lead to death of the patient. The hypotension is induced when the organ graft is connected to the vascular system of the patient and thereby reperfused. Treating such a hypotension is today an important task during organ transplantation since if the hypotension cannot be treated, there is a significant risk of patient death during the transplantation. However, by *ex vivo* treating the organ graft prior to transplantation with PEG-phospholipid molecules, the hypotension could be prevented or at least significantly inhibited or reduced. Hence, the risk of hypotension-induced damages in connection with organ transplantation is reduced by using PEG-phospholipid molecules of the invention. The PEG-phospholipid molecules of the invention are thereby useful in treating, preventing and/or inhibiting post reperfusion syndrome (PRS).

The invention thereby relates to an *ex vivo* method of treating an organ or a part of an organ. The method comprises *ex vivo* infusing a solution comprising PEG-phospholipid molecules into a vascular system and, optionally into a parenchyma, of the organ or the part of the organ. The method also comprises *ex vivo* incubating the solution comprising PEG-phospholipid molecule in the vascular system, and optionally the parenchyma, to enable coating at least a portion of the endothelial lining of the vascular system, and preferably of the parenchyma, with the PEG-phospholipid molecules while keeping the organ or the part of the organ submerged in an organ preservation solution comprising PEG-phospholipid molecules.

Thus, the *ex vivo* method comprises introducing PEG-phospholipid molecules into the vascular system of the organ or a part of the organ and therein allow the PEG-phospholipid molecules to interact with and bind to the cell membranes of the endothelium and the parenchyma. Fig. 1 schematically illustrates this principle with the PEG-phospholipid molecules hydrophobically interacting with the lipid bilayer membrane of the endothelium to thereby anchor or attach the PEG-phospholipid molecules in the cell membrane through the phospholipid group.

The interaction between the PEG-phospholipid molecules with the lipid bilayer membrane of the endothelium and optionally of the parenchyma, such as renal parenchyma in the case of a kidney, is taking place *ex vivo* while the organ or the part of the organ is submersed or submerged in an organ preservation solution comprising PEG-phospholipid molecules. Experimental data as presented herein indicates that keeping the organ submerged in an organ preservation solution comprising PEG-phospholipid molecules results in a significantly lower cascade system activation and cytokine expression as compared to merely keeping the organ or the part of the organ submerged in an organ preservation solution lacking any PEG-phospholipid molecules even if the vascular system, and optionally parenchyma, of the organ or the part of the organ is coated with PEG-phospholipid molecules.

Hence, in order to achieve an efficient *ex vivo* treatment of organs or parts of organs, the vascular system, and optionally parenchyma, of the organ or the part of the organ should be exposed to PEG-phospholipid molecules and the organ or the part of the organ should be submerged in an organ preservation solution comprising PEG phospholipid molecules. The anti-thromboinflammatory effect of such *ex vivo* treatment is significantly improved as compared to merely *ex vivo* infusing a solution comprising PEG-phospholipid molecules into the vascular system and, optionally into the parenchyma, of the organ or the part of the organ. In fact, the anti-thromboinflammatory effects of the *ex vivo* treatment of the invention were already induced during the cold ischemia period when the organ or the part of the organ is kept submerged in the organ preservation solution comprising PEG phospholipid molecules, i.e., even before reperfusion of the organ or the part of the organ.

In a particular embodiment, the organ or the part of the organ is first *ex vivo* infused with the solution comprising PEG-phospholipid molecules into the vascular system and, optionally into the parenchyma, of the organ or the part of the organ. This *ex vivo* infusion is advantageously taking place as early as possible following explanting and removing the organ or the part of the organ from the donor body. The perfused organ or part of the organ is then submerged in the organ preservation solution comprising PEG phospholipid and kept therein, preferably at reduced temperature such as about 4°C, which is further discussed herein.

In another particular embodiment, the organ or the part of the organ is first submerged into the organ preservation solution comprising PEG-phospholipid molecules and then the solution comprising PEG-phospholipid molecules is *ex vivo* infused into the vascular system, and optionally into the parenchyma, of the organ or the part of the organ. This *ex vivo* infusion can be performed while keeping the organ or the part of the organ submerged in the organ preservation solution comprising PEG-phospholipid molecules. Alternatively, the organ or the part of the organ is temporarily removed from the organ preservation solution to perform the *ex vivo* infusion and is then put back into the organ preservation solution comprising PEG-phospholipid molecules.

In an embodiment, the method also comprises *ex vivo* infusing an organ preservation solution into the vascular system to flush away non-bound PEG-phospholipid molecules from the vascular system. Hence, non-bound PEG-phospholipid molecules are preferably washed away in one or multiple, i.e., at least two, wash steps using an organ preservation solution.

Such an organ preservation solution may also be used to wash the vascular system of the organ or the part of the organ prior to infusing the solution comprising PEG-phospholipid molecules in one or multiple wash steps. In such an embodiment, the method also comprises *ex vivo* infusing an organ preservation solution into the vascular system prior to *ex vivo* infusing the solution comprising PEG-phospholipid molecules into the vascular system. The initial infusion of the organ preservation solution prior to adding PEG-phospholipid molecules will wash the vascular system of the organ or the part of the organ to thereby facilitate an efficient coating of the endothelium with PEG-phospholipid molecules.

In an embodiment, *ex vivo* infusing the solution comprising PEG-phospholipid molecules comprises *ex vivo* clamping one of an artery and a vein of the vascular system. This embodiment also comprises *ex vivo* infusing the solution comprising PEG-phospholipid molecules into the other of the artery and the vein and *ex vivo* clamping the other of the artery and the vein.

Hence, in an embodiment, a vein (or artery) of the vascular system of the organ or the part of the organ is initially clamped to prevent the solution to be added to flow out of the vascular system. The solution with PEG-phospholipid molecules is added to an artery (or vein) of the vascular system and this artery (or vein) is then clamped to prevent the solution to be added to flow out of the vascular system. Hence, the entrance and exit blood vessels of the vascular system in the organ or the part of the organ are closed by clamping to thereby keep the solution comprising PEG-phospholipid molecules within the vascular system and thereby allow PEG-phospholipid molecules to bind to the endothelium of the vascular system.

In another embodiment, the solution with PEG-phospholipid molecules is infused into an artery (or vein) of the vascular system of the organ or the part of the organ until the solution appears at a vein (or artery) of the organ or the part of the organ. This confirms that the solution with PEG-phospholipid molecules has filled the vascular system. At that point, the artery and vein are clamped.

The solution comprising PEG-phospholipid molecules can be added either through a vein or through an artery. In a particular embodiment, the solution is infused into an artery. In such a particular embodiment, the optional, initial clamping is then preferably done of a vein of the vascular system.

The clamping embodiments described above are in particular useful when the solution *ex vivo* infused into the vascular system is different from the organ preservation solution, into which the organ or the part of the organ is submerged, and/or the PEG-phospholipid molecules comprised in the solution is different from the PEG-phospholipid molecules comprised in the organ preservation solution. In other cases, no clamping is needed since the same PEG-phospholipid containing organ preservation solution will then be *ex vivo* infused as in which the organ or the part of the organ is submerged.

The solution comprising PEG-phospholipid molecules is preferably *ex vivo* incubated in the vascular system for a period of time from 10 minutes up to 48 hours to enable the PEG-phospholipid molecules to hydrophobically interact with the cell membranes of the endothelium and thereby coat at least a portion of the vascular system of the organ or the part of the organ. The *ex vivo* incubation is preferably performed from 20 minutes up to 36 hours and more preferably from 30 minutes up to 24 hours, such as from 30 minutes up to 12 hours, up to 8 hours, up to 4 hours or up to 1 hour.

The amount of solution comprising PEG-phospholipid molecules infused into the vascular system depends on the type of the organ and the size of the organ (adult vs. child). Generally, the volume of the solution should be sufficient to fill the vascular system of the organ. In most practical applications, from 5 mL up to 250 mL of the solution comprising PEG-phospholipid molecules is *ex vivo* infused into the vascular system. In a preferred embodiment, from 5 mL up to 100 mL and preferably from 5 mL up to 50 mL solution comprising PEG-phospholipid molecules is *ex vivo* infused into the vascular system.

In an embodiment, the solution comprises from 0.25 mg/mL up to 25 mg/mL PEG-phospholipid molecules. In a preferred embodiment, the solution comprises from 0.25 mg/mL up to 10 mg/mL, preferably from 0.25 mg/mL up to 5 mg/mL, such as 2 mg/mL PEG-phospholipid molecules.

The above described concentrations of PEG-phospholipid molecules can also be used for the organ preservation solution comprising PEG-phospholipid molecules.

According to the invention, the solution comprising PEG-phospholipid molecules is *ex vivo* incubated in the vascular system while keeping the organ or the part of the organ submersed or submerged in an organ preservation solution comprising PEG-phospholipid molecules. Additionally, the organ or the part of the organ is preferably also kept in a temperature above 0°C but below 8°C, preferably above 0°C but equal to or below 6°C, and more preferably above 0°C but equal to or below 4°C.

In this embodiment, the organ or the part of the organ is submerged in the organ preservation solution comprising PEG-phospholipid molecules during the incubation time when the PEG-phospholipid molecules are allowed to interact with and bind to the cell membrane of the endothelium in the vascular system. The organ or the part of the organ is preferably also kept cold, i.e., at a temperature close to but above 0°C.

According to the invention, the organ or the part of the organ is kept submerged in an organ preservation solution comprising PEG-phospholipid molecules.

It has been shown that the theoretical perfect temperature for organ preservation is 4°C - 8°C. While higher temperatures lead to hypoxic injury of the organ because the metabolism is not decreased efficiently, lower temperatures than 4°C increase the risk of cold injury with protein denaturation.

Currently, the gold standard for donor organ preservation in clinical organ transplantation uses three plastic bags and an ice box. The first plastic bag includes the organ itself immersed in an organ preservation solution. This first plastic bag is put in a second plastic bag filled with saline, and then these two plastic bacs are put in a third plastic bag filled with saline, which is then put in the ice box. More advanced organ preservation devices for keeping organs in a temperature controlled environment are available and could be used, such as the Sherpa Pak^{™} transport systems from Paragonix Technologies, Inc. Waves from Waters Medical Systems, LifePort transporters from Organ Recovery systems, etc.

The solution comprising the PEG-phospholipid molecules could be saline, an aqueous buffer solution or an organ preservation solution.

Illustrative, but non-limiting, examples of aqueous buffer solutions that could be used include phosphate-buffered saline (PBS) and a citrate solution.

The organ preservation solution that could be used to infuse the PEG-phospholipid molecules and/or wash the vascular system of the organ or the part of the organ prior to or following *ex vivo* infusing the PEG-phospholipid molecules and/or in which the organ or the part of the organ may be submerged can be selected from known organ preservation solutions. Illustrative, but non-limiting, examples of such organ preservation solutions include a histidine-tryptophan-ketoglutarate (HTK) solution, a citrate solution, a University of Wisconsin (UW) solution, a Collins solution, a Celsior solution, a Kyoto University solution and an Institut Georges Lopez-1 (IGL-1) solution.

Experimental data as presented herein shows that PEG-phospholipid molecules are readily mixable in organ preservation solutions without any precipitation (Fig. 20).

In an embodiment, the PEG-phospholipid molecules are unfunctionalized PEG-phospholipid molecules consisting of PEG-phospholipids. Hence, in this embodiment, the PEG-phospholipid molecules do not contain any functional groups. This corresponds to having R equal to CH₂ or H in Fig. 1.

In another embodiment, the PEG-phospholipid molecules are functionalized PEG-phospholipid molecules comprising a respective functionalized molecule attached to PEG of the PEG-phospholipid molecules.

In a further embodiment, the solution comprises a mixture of unfunctionalized PEG-phospholipid molecules consisting of PEG-phospholipids and functionalized PEG-phospholipid molecules comprising a respective functionalized molecule attached to PEG of the PEG-phospholipid molecules.

In the embodiments comprising only functionalized PEG-phospholipid molecules or a mixture of functionalized and unfunctionalized PEG-phospholipid molecules, the functionalized molecule is preferably selected from the group consisting of a complement inhibitor, a coagulation inhibitor, a platelet inhibitor, a molecule capable of binding a complement inhibitor, a molecule capable of binding a coagulation inhibitor, a molecule capable of binding a platelet inhibitor, and a mixture thereof.

Illustrative, but non-limiting, examples of complement inhibitors include Factor H; C4b-binding protein (C4BP); N-terminal 4 to 6 short consensus repeats (SCRs) of complement receptor 1 (CR1), also known as C3b/C4b receptor or cluster of differentiation 35 (CD35); CD46 complement regulatory protein (CD46), also known as membrane cofactor protein (MCP); and complement decay-accelerating factor (DAF), also known as CD55.

An illustrative, but non-limiting, example of coagulation inhibitors includes heparin.

An illustrative, but non-limiting, example of a platelet inhibitor is an adenosine diphosphate (ADP) degrading enzyme, such as an apyrase and ectonucleoside triphosphate diphosphohydrolase-1 (NTPDase1), also known as CD39.

Illustrative, but non-limiting examples of a molecule capable of binding a complement inhibitor is a Factor H-binding peptide, such as 5C6 disclosed in [4]; and a C4BP binding peptide, such as *Streptococcus* M protein-derived peptide M2-N, M4-N or M22-N as disclosed in [5]

An illustrative, but non-limiting example of a molecule capable of binding a coagulation inhibitor is a heparin-binding peptide, such as disclosed in [7].

Hence, in an embodiment, the functionalized molecule is selected from the group consisting of a heparin-binding peptide, N-terminal 4 to 6 SCRs of CR1, CD46, DAF, a Factor H binding molecule, an ADP degrading enzyme, and a mixture thereof.

In a particular embodiment, the functionalized molecule is selected from the group consisting of peptide 5C6, apyrase, CD39, C4BP, and a mixture thereof.

In an embodiment, the PEG-phospholipid molecules have a formula (I) or a formula (II):

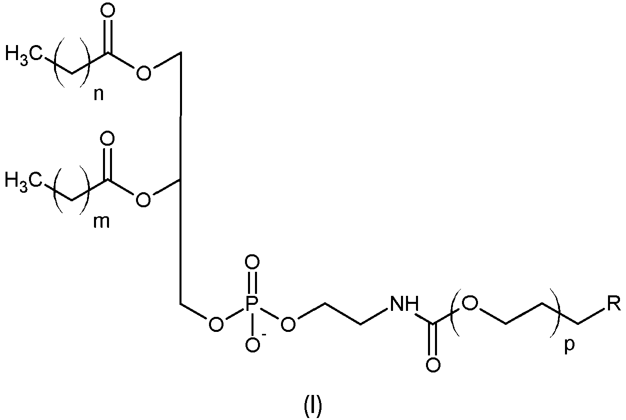

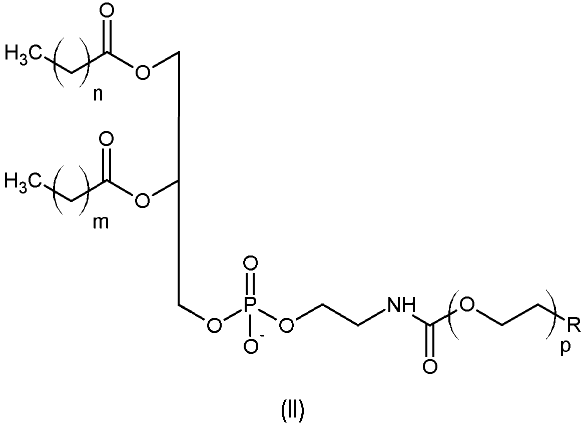

In an embodiment, n, m are integers independently selected within the range of from 10 up to 16. The parameters n, m are preferably independently 10, 12, 14 or 16, and more preferably n=m=14.

In an embodiment, p is selected so that the PEG chain has an average molecular weight selected within the range of from 1 000 Da up to 40 000 Da. The parameter p is preferably selected so that the PEG chain has and average molecular weight from 3000 up to 10 000 Da and more preferably 5000 Da.

Average molecular weight as defined herein indicates that individual PEG-phospholipid molecules may have a molecular weight different from this average molecular weight but that the average molecular weight represents the mean molecular weight of the PEG-phospholipid molecules. This further implies that there will be a natural distribution of molecular weights around this average molecular weight for a PEG-phospholipid sample.

The end group R is, in an embodiment, H, CH₂, NH₂, a malemide group, a biotin group, a streptavidin group, an avidin group or a functionalized molecule. In the case of a functionalized molecule this is preferably selected from the group consisting of a complement inhibitor, a coagulation inhibitor, a platelet inhibitor, a molecule capable of binding a complement inhibitor, a molecule capable of binding a coagulation inhibitor, a molecule capable of binding a platelet inhibitor, and a mixture thereof.

The fatty acid chains of the PEG-phospholipid molecule may be saturated. At least one or both fatty acid chains may alternatively be unsatured, i.e., comprise at least one -CH=CH- group, at least one - C≡C- group or a combination thereof. Each fatty acid chain could be straight or branched.

The PEG-phospholipid molecules *ex vivo* infused into the vascular system, and optionally parenchyma, of the organ or the part of the organ could be the same type of PEG-phospholipid molecules comprised in the organ preservation solution, in which the organ or the part of the organ is kept submerged. In another embodiment, different types of PEG-phospholipid molecules could be used in the solution *ex vivo* infused into the vascular system, and optionally the parenchyma, of the organ or the part of the organ as compared to the organ preservation solution, in which the organ or the part of the organ is kept submerged. For instance, the size of the PEG chain could be different for the PEG-phospholipid molecules with the size of the PEG chain of the PEG-phospholipid molecules selected to reach the target size in the vascular system, and optionally the parenchyma, of the organ or the part of the organ. As is shown in Fig. 19, the size of the PEG chain limits the portions of the vascular system and parenchyma, into which the PEG-phospholipid molecules can reach. In particular, 5 kDa PEG-phospholipid molecules can reach larger parts of the vascular system and parenchyma as compared to the larger 40 kDa PEG-phospholipid molecules.

Hence, in an embodiment, the PEG-phospholipid molecules comprised in the solution that is *ex vivo* infused into the vascular system and optionally parenchyma of the organ or the part of the organ may have a different average size of the PEG chain as compared to the PEG-phospholipid molecules comprised in the organ preservation solution, into which the organ or the part of the organ is submerged.

In addition, or alternatively, the PEG-phospholipid molecules in the *ex vivo* infusion solution and the PEG-phospholipid molecules in the organ preservation solution may have different function groups, i.e., end group R in formula (I) and (II).

The *ex vivo* method of the invention can be applied to any organ, or part of an organ, having a vascular system and that could be used as organ graft, i.e., transplanted into a recipient. Illustrative, but non-limiting, examples of such organs include kidney, liver, pancreas, heart, lung, uterus, urinary bladder, thymus and intestine.

In a particular embodiment, the organ is a kidney. In this particular embodiment, the *ex vivo* incubation of the solution comprising PEG-phospholipid molecules in the vascular system enables coating of not only the renal endothelium of the renal vascular system but preferably also of the tubuli of the renal parenchyma with the PEG-phospholipid molecules. Thus, experimental data as presented herein shows that the PEG-phospholipid molecules were evenly distributed in the peritubular vessels, glomeruli and the tubuli of the kidney when *ex vivo* injection a solution comprising PEG-phospholipid molecules through the aorta stump.

Experimental data as presented herein shows that PEG-phospholipid treated organ grafts had significantly less thromboinflammation as compared to untreated control organ grafts as reflected by the plasma levels of coagulation markers, such as TAT, and complement markers, such as C3a and sC5b-9. Also cytokine expression, particularly IL-6 and IL-1β, was much suppressed in the PEG-phospholipid treated organ grafts. Organ function was improved in the PEG-phospholipid treated organ grafts. PEG-phospholipid treated organ grafts also showed reduced deposition of complement markers, such as C3b and membrane attack complexes, and expression of C5aR.

Thus, *ex vivo* coating of organ grafts with PEG-phospholipid molecules protects against I/R-induced thromboinflammation, which indicates that it is a potential against I/R injury in clinical transplantation.

The disclosure, not forming part of the invention, relates to PEG-phospholipid for use in treating, inhibiting or preventing hypotension associated with reperfusion developing after reperfusion of an organ graft.

The disclosure, not forming part of the invention, relates to PEG-phospholipid for use in treating, inhibiting or preventing PRS.

A complication during and immediately, typically up to 24 hours, following organ transplantation is a significant drop in blood pressure following reperfusion of the organ graft in the recipient. This hemodynamic change in the recipient can in severe cases lead to ischemia in the organs of the recipient and in the transplanted graft causing damage to either of these entities and even death of the patient if treatment is not successful.

The observed protection against the vascular instability by the PEG-phospholipid may be due to inhibition of the contact/kallikrein system. It was unexpected found that the contact/kallikrein system was activated after reperfusion. The contact/kallikrein system generates bradykinin, a highly vasoactive peptide that induces vasodilation and increased permeability leading to hypotension, which provokes a compensatory tachycardia. Intraoperatively, the decrease in blood pressure is treated with pharmaceuticals and liquids, still a life-threatening situation may occur. Thus, a possibility to prevent the hypotension is of great clinical value.

Thus, preventing, treating or at least reducing such a hypotension and drop in blood pressure associated with reperfusion of an organ graft is a significant improvement in connection with organ transplantation. Experimental data as presented herein shows that *ex vivo* treating the organ graft prior to transplantation with a solution comprising PEG-phospholipid molecules protected the recipient against such hypotension or blood pressure drop when the organ graft was reperfused.

The PEG-phospholipid is formulated as a solution comprising PEG-phospholipid molecules and intended for *ex vivo* infusion into a vascular system of the organ graft to enable coating of endothelial lining of the vascular system and the parenchyma with the PEG-phospholipid molecules.

The disclosure, not forming part of the invention, defines use of PEG-phospholipid for the manufacture of a medicament for treating, inhibiting or preventing hypotension associated with reperfusion developing after reperfusion of an organ graft. The disclosure, not forming part of the invention, also relates to a method for treating, inhibiting or preventing hypotension associated with reperfusion developing after reperfusion of an organ graft. This method comprises the previously described method steps of *ex vivo* infusing a solution comprising PEG-phospholipid molecules into a vascular system of the organ graft and *ex vivo* incubating the solution comprising PEG-phospholipid molecules in the vascular system to enable coating of at least a portion of the endothelial lining of the vascular system with the PEG-phospholipid molecules, optionally, but preferably, while keeping the organ graft submerged in an organ preservation solution comprising PEG-phospholipid molecules.

### EXPERIMENTS

### EXPERIMENT 1

### MATERIALS, METHODS AND ANIMAL EXPERIMENTS

### Synthesis of PEG-phospholipid derivatives

### a. FITC-PEG-phospholipid

Synthesis of PEG-phospholipids was performed by methods described previously [1]. Briefly, 1,2-dipalmitoyl-*sn*-glycero-3-phosphatidylethanolamine (NOF Corporation, Tokyo, Japan, DPPE, 21 mg) was dissolved in 3 mL dichloromethane (Sigma-Aldrich Chemical Co. (St. Louis, MO, USA)). α-*N*-Hydroxysuccinimidyl-co-tert-butoxycarbonyl polyethylene glycol) (NHS-PEG-Boc, Mw: 5000 from NOF, 185 mg) and triethylamine (3 µL, Nacalai Tesque (Kyoto, Japan)) were added to the reaction solution and then stirred for 4 days at room temperature (RT, ~20°C). The NHS group of NHS-PEG-Boc readily reacted with an amino group of the phospholipids. The protective group, Boc, an amino group, was removed after the addition of 2 mL of 99 % trifluoroacetic acid (Wako Pure Chemical (Osaka, Japan)) and an additional incubation at 4°C for 30 min. The crude product was purified by re-precipitation in diethyl ether (Nacalai Tesque). After extraction with chloroform (Nacalai Tesque) and evaporation, the PEG-phospholipids were obtained as white solids. The yield was 85 %. For fluorescent labeling with fluorescein isothiocyanate (FITC) (Dojindo Laboratories (Kumamoto, Japan)), PEG-phospholipids (200 mg) were allowed to react with FITC (31 mg) in acetone for 12 h. FITC-PEG-phospholipid was purified by gel permeation chromatography (Sephadex G-25, GE Healthcare (Buckinghamshire, UK)).

The synthesis of FITC-PEG-phospholipid (40 kDa) was according to the following. α-N-hydroxysuccinimidyl-ω-maleimidyl poly(ethylene glycol) (NHS-PEG(40k)-Mal, Mw: 40,000) and DPPE (9.6 mg) were dissolved in 3 mL dichloromethane, and triethylamine (3 µL, Nacalai Tesque) were added to the reaction solution and then stirred for 4 days at room temperature (RT, ~20°C). The crude product was purified by re-precipitation in diethyl ether. After extraction with chloroform and evaporation, the Mal-PEG-phospholipids (40 kDa) were obtained as white solids. The yield was 80 %. For fluorescent labeling with fluorescein isothiocyanate (FITC), Mal-PEG-phospholipids (40 kDa) (1200 mg) was dissolved in DMSO (Nacalai Tesque, 20mL), and then FITC-glycine-cysteine (FITC-GC, BEX Co., Ltd., Tokyo, Japan, 12 mg) was added and incubated for 24 hr at 37°C. The crude product was purified by re-precipitation in diethyl ether. After extraction with chloroform and evaporation, the FITC-PEG-phospholipids (40 kDa) were obtained as yellow solids

### b. Biotin-PEG-phospholipid

Biotin-PEG-phospholipid was synthesized by combining polyethylene glycol) (*N*-hydroxysuccinimide 5-pentanoate) ether 2-(biotinylamino)ethane (biotin-PEG-NHS, Mw: 5000 from NOF, 180 mg) and 1,2-dipalmitoyl-*sn*-glycero-3-phosphatidylethanolamine (DPPE) (20 mg) with triethylamine (50 µL) and dichloromethane (4 mL) and stirring for 48 h at RT. Precipitation with diethyl ether yielded biotin-PEG-phospholipid as a white powder (165 mg; 80 % yield). Biotin-PEG-phospholipid was used for the visualization without further purification.

### c. PEG-phospholipid

PEG-phospholipid was synthesized by combining α-succinimidyloxysuccinyl-ω-methoxy, polyoxyethylene (MeO-PEG-NHS, Mw: 5000 from NOF, 171 mg) and DPPE (22 mg) with triethylamine (25 µL) and dichloromethane (5 mL) and stirring for 72 h at RT. Precipitation with diethyl ether yielded PEG-phospholipid as a white powder (160 mg; 80 % yield). PEG-phospholipid was used for kidney treatment without further purification.

### Functional evaluation of various types of PEG-phospholipids

In order to evaluate whether the functional moiety had any impact on its protective properties, a series of experiments were made on primary human mesenchymal stem cells (hMSCs). hMSCs were coated with PEG-phospholipid, FITC-PEG-phospholipid and biotin-PEG- phospholipid. After the surface modification, viability of the coated cells was analyzed by Allmar blue assay (Life technologies, Stockholm; Sweden) according to the manufacturer's instructions. Briefly, coated cells were cultured in MSC culture media in a 96 well cell culture microplate. After 6 hours culture, 10 µL of Allmar blue reagent was added to 100 µL of culture media followed by 4 hours incubation at 37°C. Subsequently, the fluorescence emission was measured at 580-610 nm.

To determine the hemocompatibility of the hMSC, surface-modified MSCs were exposed to whole blood in a loop model as previously described [7]. In summary, whole blood was drawn from healthy volunteers in heparin-coated tubes (Corline AB, Uppsala; Sweden). Heparinized PVC tubing (30 cm) was used. Each tubing was filled with 3 mL of blood to which 10,000 MSC/mL was added. The tubing was rotated at a speed of 30 rpm at 37°C for 60 min. After incubation, 1.2 mL of blood was collected in 2 mL tubes (Eppendorf, Germany) containing EDTA at a final concentration of 10 mM. Subsequently, blood was centrifuged at 4200×*g* for 15 min at 4°C to obtain plasma. The plasma was divided into aliquots and stored at-80°C until further analyses.

### Retention of PEG-phospholipid with different length of the PEG subunit

Cells (CCRF-CEM, floating cell line; n=3) were incubated with FITC-PEG-phospholipid (2 mg/mL in PBS) for 30 min at RT, followed by washing with culture medium. Here, FITC-PEG-phospholipids with different molecular weight of PEG chain, 1 kDa, 5 kDa and 40 kDa, were used. The treated cells were cultured in culture medium at 37°C, and collected for flow cytometry analysis at 0, 1, 3, 6, 24, and 48 hr.

### Porcine allogeneic transplantation model

The pig was chosen as a large animal model because of its anatomy and physiology, which closely resemble that of human beings. Specifically, the structure and function of the kidney is very similar to the primate organ. In all studies on pigs, conventional breeds with a high health standard were used. In the long-term survival experiments the animals were acclimatized for a two-week period and underwent a training and socialization program. Clinical examinations, including ultrasound of the urinary tract, hematology, and clinical pathology were frequently performed, and SLA-typing in order to mismatch donors and recipients was undertaken before transplantation. The recipient pigs recovered quickly after surgery but were not treated with immunosuppressive pharmaceuticals. Administration of such drugs interfere with the immune response and was excluded in the experiments. The pigs were therefore euthanized after 96 h in order to avoid acute rejection of the transplant. A full necropsy was performed after euthanization. All studies were approved by the ethics Committee for Animal Experimentation, Uppsala, Sweden.

### A. Short-term model (perfusion of the kidney with PEG-phospholipid alone for 40 min)

Ischemia reperfusion injury (I/R injury) is a major contributor to the pathology of several conditions including transplantation, thrombotic disorders, sepsis and cardiopulmonary bypass. The present experiment tested whether *ex vivo* coating of ischemic cell surfaces of porcine kidney grafts with PEG-phospholipid protects against I/R injury in an en-bloc allogeneic kidney transplantation model.

*Animals.* The study was performed at Hedenstierna experimental facility, Uppsala University Hospital, Sweden. Six donors and six recipient pigs of both sexes were included. Weight and age of the pigs varied between 30-35 kg and 10-12 weeks, respectively. Non-survival procedures were applied and animals were sacrificed after a post-transplant observational period of 8 hours.

*Anesthesia.* Upon arrival at the laboratory, an intramuscular injection of 2.2 mg/kg xylazine (Rompun; Bayer, Leverkusen, Germany) in combination with zolazepam 6 mg/kg (Zoletil; Virbac, Carros, France) were given for sedation. Subsequently, the animals were secured in a prone position. Peripheral venous catheters were introduced in both ear veins for induction and maintenance of anesthesia and for fluid administration. An epitracheal mini-incision was made for exposure of trachea and transtracheal intubation for mechanical ventilation Servo-IMechanical Ventilator, MAQUET, Medical Systems, US. The animals were ventilated with 30% oxygen in air to obtain 5.0-5.5 kPa CO₂. A mean arterial pressure (MAP) of > 60 mmHg was aimed to insure sufficient organ perfusion.

*Transplant model.* We developed a porcine transplant model enabling a dual en-bloc retrieval and implantation of both kidneys from a donor into a recipient animal. By isolated *ex vivo* PEG-phospholipid incubation of just one randomly selected kidney within the en-bloc package, this technique allowed us to co-transplant the treated organ with its perfectly matched control tissue in one recipient pig, which not only reduced the number of experimental animals but also minimized experimental confounding variables.

The trunk of the supra- and infrarenal aorta as well as the inferior vena cava were mobilized so an en-bloc package was created consisting of the two kidneys, ureters and the complete renal vasculature. The en-bloc package was immediately removed and cold flushed with HTK (Custodiol; Köhler Chemie GmbH, Germany) solution. Thereafter, the en-bloc package was cold stored in HTK solution at 4°C for 24 hours.

Post-preservation, one kidney within each en-bloc package was randomly selected for PEG-phospholipid incubation. The vein and the artery of the contralateral kidney (control) as well as the vein of the selected kidney were clamped. A total of 10-15 mL of PEG-phospholipid solution with a concentration of 2 mg/mL was slowly infused in the selected kidney. The artery of the treated kidney was then clamped and the en-bloc package was cold stored for another 40 min at 4°C in HTK. Post-incubation and before the implantation, the redundant PEG-phospholipid flushed from the treated-kidney with HTK without removing the clamps from the control.

The recipient pigs were handled and anesthetized as previously described. The en-bloc package was placed horizontally intra-abdominally. The distal end of vena cava and aorta of the transplant was anastomosed end-to-side to the recipient's cava and aorta, respectively. Upon de-clamping, the treated and control kidney were sequentially reperfused by delayed de-clamping of the control kidney with seconds in between, while the effluent from the treated kidney was flushed out. This was done in order to prevent contamination of the control kidney from the remaining PEG-phospholipid solution within the aortic conduit. Both transplant ureters were separately catheterized for documentation of urinary output.

### B. Long-term model (perfusion of the kindey with PEG-phospholipid alone for 40 min)

*Animals.* Based on the results from SLA typing donor (n=12) and recipient (n=21) crossbred pigs were chosen for mismatch transplantation. The animals were housed in individual pens measuring 3 m², within sight and sound of one another. Straw and wood shavings were used as bedding. A 14:10 h light/dark schedule (lights on at 06:00 h) was applied and an infrared lamp (24 h) was provided in each pen. The temperature was 16-18°C. The animals were fed commercial finisher diet without growth promoters (SOLO 330, Lantmannen, Sweden) twice daily, the amount depending on BW and according to the Swedish University of Agricultural Sciences regimen for growing pigs. Water was provided ad *libitum.* Donor pigs were anaesthetized with a combination of medetomidine (Domitor^{®} vet, 1 mg/mL; Orion Pharma Animal Health, Sollentuna, Sweden) at a dose of 0.1 mg/kg BW, butorphanol (Butomidor vet, 10 mg/mL; Salfarm Scandinavia, Helsingborg, Sweden) at a dose of 0.2 mg/kg BW and ketamine (Ketaminol^{®} vet, 100 mg/mL; Intervet International BV, Boxmeer, Netherlands) at a dose of 5 mg/kg BW intramuscular (i.m.) and kidneys were examined with ultrasound (Logiq e R6, GE Healthcare, Wauwatosa, U.S.A.) using linear (10 MHz) and curvilinear (4 MHz) probes to exclude pigs with renal cysts. Kidney length, echogenicity and cortiocomedullary definition were assessed, the renal pelvic region was evaluated for evidence of pyelectasia and color Doppler was used to evaluate for the presence of blood flow in the kidneys. During a two week acclimatization period the pigs were handled daily and trained for different examinations to be performed without restraint after the transplantation.

*Transplantation model.* Anesthesia was induced with a combination of medetomidine (Domitor^{®} vet, 1 mg/mL; Orion Pharma Animal Health, Sollentuna, Sweden) at a dose of 0.05 mg/kg BW, and tiletamine and zolazepam (Zoletil^{®}, 50 mg + 50 mg/mL; Virbac, Reading, Carros, France) at a dose of 5 mg/kg BW i.m. Buprenorphine (Vetergesic^{®} vet, 0.3 mg/mL; Orion Pharma Animal Health, Sollentuna, Sweden) at dose of 0.01 mg/kg BW intramuscularly and epidural morphine (Morfin Epidural Meda, 2 mg/ml; Meda, Solna, Sweden) at a dose of 0.1 mg/kg were given preoperatively for additional analgesia. Procaine benzylpenicillin (Penovet^{®} vet, 300 mg/mL; Boehringer Ingelheim Vetmedica, Ingelheim am Rhein, Germany) was given pre-operatively at a dose of 20 mg/kg BW i.m. After anesthesia induction vein catheters for long-term use (CareflowTM, 3Fr, 200mm, BD, Franklin Lakes, NJ, USA) were inserted into *vena auricularis* by Seldinger technique. Post-operatively, the catheters facilitated stress free blood sampling. Catheters were flushed once daily with heparinized saline 100 lE/mL (Heparin LEO, 5000 lE/mL; Leo Pharma, Ballerup, Denmark). Anesthesia was maintained with isoflurane (IsoFlo^{®} vet; Orion Pharma Animal Health, Sollentuna, Sweden) vaporized in oxygen. The pigs received fluid therapy with crystalloids (Ringer-acetat; Fresenius-Kabi, Bad Homburg, Germany) continuously. Colloids (Gelofusine^{®} 40 mg/mL; Braun, Melsungen, Germany) and dobutamine (Dobutamin Carino, 250 mg/mL; Carinopharm, Elze, Germany) were given when needed to maintain blood pressure within reference values. During anesthesia, respiratory and cardiovascular parameters were monitored.

*Donor procedure.* In a first series of experiments (3+3 donors and 6+5 recipients), both kidneys were procured, similarly treated with HTK perfusion (n=11), kept on ice overnight and then one of the kidneys was randomly treated with PEG-phospholipid 60 min before insertion whereas the other kidney was left untreated. An alternate administration of PEG-phospholipid was applied in another ten kidneys (5 donors and 10 recipients) which were either treated with HTK perfusion containing PEG-phospholipid or regular HTK perfusion solution. After the procedure, donor pigs were euthanized by i.v injection of pentobarbital sodium (Allfatal vet, 100 mg/mL; Omnidea AB, Stockholm, Sweden) under general anesthesia.

*Transplantation surgery.* The recipient pigs underwent allogeneic single kidney transplantation. All procedures were performed intra-abdominally through a 15-20 cm midline incision, and the iliac vessels in right iliac fossa were identified and gently mobilized. The distal segment of the vena cava as well as the iliac artery (from its emergence from the aorta) were mobilized sealing the surrounding lymphoid tissue. The renal graft was thereafter placed in the right iliac fossa in proximity to the iliac vessels. The renal vein and artery were trimmed and subsequently anastomosed in an end-to-side fashion to the recipient's right iliac artery and distal vena cava using a polypropylene 7/0 running suture (PROLENE^{®}, Ethicon, U.S.). The ureters were implanted by extravesical ureteroneocystostomy to the top of the bladder by 6-0 polydioxanone suture (PDS^{®}, Ethicon, US). Thereafter, a bilateral nephrectomy of the native kidneys was performed. The midline incision was closed by running 2/0 polyglactin (VICRYL^{®} Ethicon, US) fascia suture and skin clips. The mean duration of the surgery was 3 h (2.5 - 5.5h).

Towards the end of surgery, isoflurane administration was discontinued, the fraction of inspired oxygen was increased (FiO₂ 1.0) and the pigs were weaned off the mechanical ventilator and spontaneous breathing was restored. The pigs were placed in an intensive care cage where they could easily be monitored continuously by the staff. Oxygen supplementation was provided and heart rate, respiratory rate, oxygen saturation and body temperature were monitored until the pigs were conscious. During the first day, the pigs were hand-fed fruits and supported to drink water if needed. They were also supported to stand and walk and received massage over gluteus medius, biceps femoris and gluteus maximus several times. For postoperative analgesia, buprenorphine was administered i.v. during two days.

*Euthanasia and necropsy.* The recipients were euthanized with an overdose of pentobarbital sodium (Allfatal vet, 100 mg/mL; Omnidea AB, Stockholm, Sweden) after 96 h. Kidney biopsies for histological examinations were collected immediately post mortem and all pigs underwent post mortem examinations at the Department of Biomedical Sciences and Veterinary Public Health, Division of Pathology, SLU.

*Pre-treatment of HTK solution.* In another set of experiments, four pigs were transplanted with PEG-phospholipid treated kidneys and five pigs with control kidneys. In this set of experiments, after procurement, the kidneys were perfused with HTK (Custodiol; Köhler Chemie GmbH, Germany) solution with 2 mg/mL of PEG-phospholipid (treated kidneys) or without PEG-phospholipid (control kidneys). The kidneys were also stored submerged in the HTK solution with 2 mg/mL of PEG-phospholipid (treated kidneys) or in the HTK solution without any PEG-phospholipid (control kidneys) at 4°C for 24 hours.

### C. Long term model for evaluation of detachment of PEG-phospholipid from the kidney endothelium

Kidneys were procured from donor pigs and transplanted into recipient pigs after two weeks acclimatization and training as described above. The kidneys were treated using the same procedure as for those transplants which received PEG-phospholipid 60 min before insertion. In order to measure detachment from the endothelium the PEG-phospholipid was fluorescence labelled. The same anesthetic and surgical protocol were used but the native kidneys were left in the recipients (n=4). The pigs were euthanized after 12, 24, 48 and 72 hours, respectively. All pigs underwent a necropsy and biopsied were collected for histology and monitoring of fluorescence.

### Blood sampling

In the short-term model, blood samples were obtained from the left and right kidney transplant veins at 5, 15, 30, 60, 120, 240 and 360 minutes after start of the reperfusion. In the long-term model blood was obtained after 1, 24, 48, 72 and 96 hours. The samples were drawn into EDTA Vacutainer tubes (Becton Dickinson AB, Sweden) and immediately centrifuged at RT for 15 minutes. The collected plasma was stored at -80°C for further analysis.

### TAT, C3a and sC5b-9 ElAs

TAT, C3a, and sC5b-9 in plasma were measured by sandwich ElAs as previously described [7]. In short, for the assessment of TAT, plasma samples were diluted 1/100 in normal citrate phosphate dextrose (CPD) plasma. An anti-human thrombin monoclonal antibody was used for capturing and a HRP coupled anti-human antithrombin antibody (AT) was used for detection (Enzyme Research Laboratories, South Bend, IN, USA). Standard was prepared by diluting pooled human serum in normal CPD plasma. Values were expressed as µg/L. For assessment of C3a, plasma was diluted 1/1000 in working buffer (PBS containing 0.05% Tween 20, 10 mg/mL BSA, and 10 mM EDTA). Anti-human C3a mAb 4SD17.1 was used for capturing C3a and biotinylated polyclonal rabbit anti-C3a antibody followed by HRP-conjugated streptavidin for detection of plasma C3a. Zymosan-activated serum, calibrated against purified C3a, served as a standard. Values were expressed as ng/mL. sC5b-9, plasma was diluted 1/50 in working buffer (as above). sC5b-9 was captured by anti-human C5b-9 mAb (a9e) and detected by anti-human C5 polyclonal rabbit antibody (Dako) and HRP-conjugated anti-rabbit IgG (Dako). Zymosan-activated serum containing 6x104 AU/mL was used as standard. Values were expressed as AU/mL.

### Histological analyses

Kidney tissue biopsies were taken from control and biotin-PEG-phospholipid treated kidney at 5, 60 and 360 minutes post reperfusion. For the detection of PEG-phospholipid the biopsies were immediately frozen in liquid nitrogen and stored at -80°C until further analyses. Frozen sections (4 µm thickness) of kidney treated with biotin-PEG-phospholipid were incubated in Alexa488-streptavidin (GE Healthcare, 1:500) at RT for 10 min. The sections were analyzed by laser-scanning confocal microscopy (LSM510, META, Carl Zeiss, Germany). Both biotin-PEG-phospholipid and FITC-PEG-phospholipid (5 kDa, 40 kDa) were assessed.

Tissue samples were also fixed in 4% paraformaldehyde solution overnight and stored in 70% ethanol until embedded in paraffin. The paraffin sections (5 µm thickness) were de-waxed in xylene and rehydrated with ethanol. Heat-induced antigen retrieval was performed by boiling the sections twice in sodium citric buffer. Blocked with goat sera before primary antibody incubation. The sliced sections were stained with hematoxylin and eosin (HE staining), and antibodies for complement markers such as C5aR (Acris Antibodies, Herford, Germany), C3b (Bioss Antibodies, Woburn, MA, USA), C4d (Abcam, Cambridge, UK) and C5b-9 (MAC; Abcam). As a control experiment, IgG rabbit (Dako, Glostrup, Denmark) and IgG mouse (Abcam) were used. Primary antibody detection was performed with Dako real Detection System alkaline phosphatase red (Dako) followed by counterstaining with hematoxylin. Slides were visualized using a Zeiss Axio Imager A1 microscope. The lens was a 10X objective and the fields evaluated were 800,000 µm². The square regions were quantified for intensity and area of the immune reactive regions using the Axio Visio (rel. 4.8) Software (Zeiss, Jena, Germany). Results are presented as mean densitometric sum red. Histological analysis was performed by a pathologist double blinded to samples for detection of tubular dilatation and necrosis, glomerular injury and vacuolization.

### Statistical analysis

Results are presented as means ± SEM. Data plotting and statistical analysis were performed using Prism version 6 for Macintosh software (Graphpad, San Diego, CA, USA). Differences between means of two groups were statistically evaluated using Wilcoxon paired test whereas statistical significance between more than two groups was analyzed by repeated measure 2-way ANOVA followed by post Hoc Bonferoni evaluation. Comparison between groups is considered to be significant when p<0.05, while values above were considered as non-significant (ns).

### Results

### Comparison of the functional properties of the various PEG-phospholipid preparations

hMSCs were treated with the various types of PEG-phospholipid preparation. Viability of the hMSCs was unaltered post-surface modification with PEG-phospholipids alone (97.3%), FITC-labelled PEG-phospholipids (98.3%) as well as with biotin-labeled PEG-phospholipid (97%) indicating that PEG-phospholipids do not affect viability.

The retention of PEG-phospholipid with different length of the PEG subunit was tested using CCRF-CEM floating cells. The cells were coated with PEG-phospholipid with 1 kDa, 5 kDa and 40 kDa PEG subunits conjugated with FITC. The cells were incubated at 37°C and the binding was monitored by flow cytometry. It was shown that the binding of all preparations was lost 90% after 24 hrs, but the 5 kDa preparation was the one that was retained the longest time (Fig. 13).

TAT levels were significantly lowered when PEG-phospholipid coated cells compared to uncoated cells were exposed to whole blood. This reduction in TAT generation was similar for cells coated with either PEG-phospholipid alone (61.5 ± 2.9 vs. 110 ± 7.6), FITC-labeled PEG-phospholipid (60.1 ± 2.7) or with biotin-labeled PEG-phospholipid (61.4 ± 5.1) when compared with controls without PEG-phospholipid (110 ± 7.6) coating (Fig. 2A).

A similar trend was observed for the complement activation markers when coated and un-coated cells were exposed to whole blood. At 60 min after blood exposure, the plasma levels of C3a and sC5b-9 were significantly reduced if cells were coated with PEG-phospholipid (50.8 ± 3.2, 37.7 ± 1.5), FITC-labeled PEG-phospholipid (51.3 ± 3.8, 35.0 ± 2.1), and biotin- labeled PEG-phospholipid (49.8 ± 4.1, 38.3 ± 2.0) in comparison with uncoated cells (104 ± 7.7, 104 ± 6.2) (Figs. 2A and 2B).

### Distribution of FITC-labelled phospholipid in pig kidneys treated with PEG-phospholipids

Allogeneic kidneys were transplanted en-bloc to recipients (n=6) as described in materials and methods. The distribution of PEG-phospholipid in the kidney tissue post reperfusion in the recipient was analyzed in biopsies taken at 5, 60 and 360 min. As shown in Fig. 4, the fluorescence before reperfusion from FITC-labelled PEG-phospholipid was evenly distributed in the peritubular vessels, glomeruli and the tubuli of the whole kidney, reflecting that the kidney tissue was equally modified with PEG-phospholipid via the injection of PEG-phospholipid solution through the aorta stump. By contrast, there was no fluorescence in the control kidney tissues, indicating that there was no significant leakage between the test and control kidneys.

When kidney tissues at 5, 60 and 360 min were compared, the fluorescence of, particularly, the glomeruli but also the peritubular vessels and the tubuli, in the FITC-labelled PEG-phospholipid-treated kidney tissues gradually decreased with time, which indicated that PEG-phospholipid detached from the cells albeit that there was still some PEG-phospholipid bound to the tubules at 360 min.

In the long-term study for evaluation of detachment (C. Long term model for evaluation of detachment of PEG-phospholipid from the kidney endothelium), only minor fluorescence was visible after 24 h and after 48 h no fluorescence was observed which shows that all PEG-phospholipid had detached. *Ex vivo* FITC-labelled PEG-phospholipid also attached to the endothelium of cardiac vessels.

### A. The short-term allogeneic transplantation model

### Immunohistochemical analysis of the transplanted kidneys

Transplanted kidneys were analyzed by immunohistochemistry for albumin leakage, complement deposition and receptor expression (C3a, sC5b-9, C4d, C3b; Figs. 5A to 5D). Histological evaluation revealed lower levels of deposition of complement activation markers such as C3b/iC3b and C5b-9 and expression of complement receptor C5aR in PEG-phospholipid treated kidney grafts compared to the control kidneys. There was a significant reduction in the C3b/iC3b deposition at 5 min in PEG-phospholipid treated kidney post perfusion. For albumin, or the oxidative stress markers HO-1, iNOS, and nitrotyrosine, there was a tendency (ns) that those markers were lower in PEG-phospholipid treated kidneys compared to the control kidneys. Thus, the results from the histological ranking support that surface treatment of the kidney graft by PEG-phospholipid protects against complement activation and that there is a tendency to suppressed oxidative stress.

### Coagulation and complement activation

In order to assess the influence of the PEG-phospholipid treatment on the vascular innate immune system after reperfusion of the kidneys in the short-term porcine en-bloc allogeneic transplantation model, coagulation activation (TAT, Fig. 6A) and complement activation markers (C3a, sC5b-9, Figs. 5A and 5B) in blood were analyzed. TAT levels were lower in blood of recipients with PEG-phospholipid treated kidneys compared to control recipients. A significant reduction in TAT levels in PEG-phospholipid treated kidneys versus uncoated controls was observed at 5 min (46.1±5.2 vs. 73±12.3, p: 0.01), 15 min (48±8.4 vs. 73±9, p: 0.02), 30 min (41±9.1 vs. 57±4.9, ns), 60 min (32±4.8 vs. 58±3.7, p: 0.02), and 120 min (28.6±5.1 vs. 53±4, p: 0.02), but not after 240 min (32±4 vs. 49±6.1, ns) and 360 min (29±9 vs. 41±14, ns) post reperfusion. These results indicated that coagulation activation was significantly suppressed by the surface modification after kidney transplantation.

Similar results were observed for the complement markers (C3a and sC5b-9). There was a significant reduction in C3a levels in the blood of recipients' transplanted with PEG-phospholipid-treated kidneys compared to the control recipients after 5 min (40±2.8 vs. 63.6±5.4, p: 0.03), 15 min (39±4 vs. 61±7.2, p: 0.04), 60 min (41±5.2 vs. 68±5.1, p: 0.01), 120 min (44±6.1 vs. 67±3.1, p: 0.03), 240 min (45±12 vs. 93±3, p<0.0001) and 360 min (45±12 vs. 80±16, p<0.001), but not at 30 min (39±5.8 vs. 54±5.9, ns) post reperfusion. In addition, a significant reduction in sC5b-9 level was also observed compared to control recipients. Generation of sC5b-9 in samples from the treated kidneys was reduced at all time points, except after 5min, in comparison with the controls. The plasma levels of sC5b-9 at 5 min after reperfusion of treated and control kidneys were (16±6 vs. 31±5, p: 0.2), after 15 min (15±3 vs. 45±11, p: 0.01), after 30 min (16±4 vs. 45±3, p: 0.01), after 60 min (25±7 vs. 54±4, p: 0.01), after 120 min (36±12 vs. 64±9, p: 0.01), after 240 min (45±11 vs. 75±11, p: 0.01), and after 360 min (44±14 vs. 79±11, p=0.008). Thus, these results showed that complement activation was significantly suppressed by coating the endothelium with PEG-phospholipid.

### Kidney function

Kidney function was analyzed by evaluating the diuresis after kidney transplantation with and without PEG-phospholipid treatment (Fig. 10A). There was a significant increase in the total urine production from the recipients with PEG-phospholipid treated kidneys compared to the control recipients at 360 min post reperfusion (48.3±22.2 mL vs. 33.5±18.2 mL, p<0.05). However, there were no significant differences between groups with regard to diuresis time or the levels of serum creatinine since the recipients' kidney were not removed.

### B. The long-term allogeneic transplantation model

### Coagulation and complement activation

In order to assess the influence of the PEG-phospholipid treatment on the vascular innate immune system after reperfusion of the kidneys in the long-term porcine allogeneic transplantation model, complement activation markers (C3a, sC5b-9, Fig. 8A and 8B) and coagulation activation (TAT, Fig. 8C) in blood were analyzed. TAT levels were lower in blood of recipients with PEG-phospholipid treated kidneys compared to control recipients. A significant reduction in TAT levels in PEG-phospholipid treated kidneys versus uncoated controls was observed at 24 hour (372±123 vs. 137±21, p<0.05), 48 hour (365±82 vs. 152±17, p<0.05), 72 hour (467±96 vs. 135±18, p<0.001), and 96 (428.6±111 vs 168±23, p<0.01), but not after 1 hour (378±82 vs. 176±24, ns) post reperfusion. These results indicated that coagulation activation was significantly suppressed by the surface modification after kidney transplantation.

Similar results were observed for the complement markers (C3a and sC5b-9). There was a significant reduction in C3a levels in the blood of recipients' transplanted with PEG-phospholipid-treated kidneys compared to the control recipients after 48 hours (119±40 vs. 67±9, p<0.001), 72 hours (95±13 vs. 63±10, p<0.01) but not after 1 hour (68±11 vs. 54±4, ns), 24 hours min (128±54 vs. 63±18, ns) or 96 hours (103±21 vs. 74±8, ns), post reperfusion. In addition, a significant reduction in sC5b-9 level was also observed compared to control recipients. Generation of sC5b-9 in samples from the treated kidneys was reduced at all time points, except after 5 min, in comparison with the controls. The plasma levels of sC5b-9 at 5 min after reperfusion of treated and control kidneys were 24 hour min (73±29 vs. 33±16, p<0.05), 48 hour (94±33 vs. 33±11, p<0.001), 72 hour (126±46 vs. 41±18, p<0.0001), and 96 (108±23 vs. 51±11, p<0.01) but not after 1 hour (63±11 vs. 33±16, ns). Thus, these results showed that complement activation was significantly suppressed by coating the endothelium with PEG-phospholipid.

### Proinflammatory cytokines

The concentration of cytokines/chemokines in the long-term allogeneic kidney transplantation were assessed. Using a multiplex assay, the concentration in plasma of INFγ, IL-10, IL-2, IL-1α, IL-1R, IL-4, IL-6, IL-10, IL-18, IL-8, IL-12, and TNF were assessed, see Figs. 9A to 9L. The levels of IL-10, IL-1R, IL-4, IL-6, IL-12, IL-18 and TNF were significantly lower in the animals transplanted with the PEG-lipid treated kidneys after 4 days.

### Kidney function

All animals recovered well after transplantation and started to eat within 24 h. Urine production was confirmed with ultrasound examination and urine was found in the bladder within 72 h in all pigs. Kidney function was analyzed by assessing the creatine levels after kidney transplantation with and without PEG-phospholipid treatment (Fig. 10B). There was a significantly lower level in the recipients with PEG-phospholipid treated kidneys compared to the control recipients over the whole four day period.

### Necropsy

No signs of disease apart from the changes related to the renal transplantation were observed. The post mortem examinations were performed at the Section of Pathology, SLU, Uppsala.

### Post perfusion syndrome

In our long-term survival pig studies it was shown that the animals, which were transplanted with PEG-phospholipid treated kidneys, had minimal or no changes of the heart rate and arterial blood pressure after reperfusion. Thus, the PEG-phospholipid product also prevented activation of the contact system and ultimately PRS, see Fig. 11.

### Pre-treatment of HTK solution

Figs. 16A and 16B show the results of evaluating incubation of the pig kidneys for 24 hours in PEG-phospholipid supplemented HTK solution at 4°C. The TAT values are presented in Fig. 16A, and the plasma creatinine values are presented in Fig. 16B. There was a significant improvement in keeping the pig kidneys in the HTK solution complemented with PEG-phospholipid as compared to the control with only HTK solution and no PEG-phospholipid treatment. The low plasma creatinine levels seen in the treated pig kidneys were obtained even without any added immunosuppression.

### Organ storage

When PEG-lipid was dissolved in perfusion liquid in combination with long-lasting storage of the graft at +4°C, there were significantly lower cascade system activation (Figs. 17A to 17D) and levels in the expression of cytokines (Figs. 18A to 18G) in the PEG-lipid treated kidneys upon reperfusion.

### Histological analysis of FITC-PEG-phospholipid

Fig. 19 illustrates confocal microscopic images of biopsies from kidneys treated with FITC-PEG-phospholipid (5 kDa) and FITC-PEG-phospholipid (40 kDa) together with control. These images thereby show the uptake of 5 kDa PEG-lipid and 40 kDa PEG-lipid after *ex vivo* infusion of PEG-lipids into the kidneys from four different pigs where one kidney was used for the 5 kDa PEG-lipid and the other for 40kDa PEG-lipid. As is shown in Fig. 19, the 5 kDa PEG-lipid penetrated more efficiently into the parenchyma of the kidney as compared to the 40 kDa PEG-lipid. Accordingly, the 40 kDa PEG-lipid molecules were less efficiently filtered through the glomeruli of the nephrons than the 5 kDa PEG-lipid molecules.

### Discussion

In many diseases and pathological conditions, ischemia induces the pathological changes. In principal, four types of mechanisms induce ischemia in the tissue namely, thrombosis, transplantation, clamping of the artery leading to an organ and sepsis. IRI is an inevitable event during organ transplantation but it also occurs in a wide range of other clinically relevant situations such as brain death, sepsis, cardiac surgery as well as after reperfusion of the ischemic area in threatening myocardial infarction or stroke. In the present experiment we demonstrate that coating of the endothelial lining and the tubular system with a PEG-phospholipid construct prior to transplantation in both a short-term and a long-term porcine allogeneic transplantation model, functionally replaces the GXC and strongly attenuates the initial I/R injury response *in vivo.*

The transplanted kidney is exposed to ischemic injury under various stages from the time period in the donor to the actual transplantation event. Since cadaveric donors serve as the main contributors of organ donation, hemodynamic instability of the circulation in cadaveric donors is one of the main causes of ischemia. Warm ischemia is experienced during organ procurement from the cadaveric donors and cold ischemic injury occurs during organ preservation at lower temperature. Reperfusion of the organ in the recipient finalizes the I/R injury.

In order to overcome these issues, we present a novel method of local protection from innate immune attack in kidney transplantation. Kidney endothelium and tubules was coated with PEG-phospholipids by hydrophobic interactions with cell lipid bilayer membrane, thereby replacing the GXC layer. The PEG chains are inert and provide high biocompatibility. Hence, the PEG-phospholipid alone protects against I/R injury by shielding off the endothelial and tubular cell membranes and by hindering proteins to reach and become activated on the endothelial cell surfaces of the kidney.

The PEG-phospholipid treated kidneys in recipients showed less immune activation as reflected in attenuated plasma levels of TAT, FXlla, TF, C3a and sC5b-9, and deposition of C3b on the endothelial surface of the kidney grafts. This showed that PEG chains sticking out of the lipid bilayer membrane could inhibit complement activation and protect the cell membrane from deposition of complement components after reperfusion of fresh blood from the recipients. It also resulted in the suppression of coagulation activation. Complement and coagulation activation is the hallmark of I/R and the reduced levels of the complement markers corroborate the concept of a sheltering effect against I/R injury leading to rejection.

In the short-term study, the transplant was followed for up to 6 hours for evaluation of kidney function and local hemocompatibility. When the PEG-phospholipid suspension was injected via the common aorta segment of the two kidney grafts, the tissue of the whole test kidney was equally coated with PEG-phospholipid including the glomeruli (Figs. 4B, 4D, 4F). The gross morphology of the kidney grafts was unchanged indicating that the treatment of the kidney graft with PEG-phospholipid is without adverse effects on cell viability. Also, when the total urine production was compared between control kidney and PEG-phospholipid-treated kidney, a significant improvement of diuresis was observed in the test kidney. These results indicate that not only the kidney endothelium was protected from the innate immunity attack, but also that the kidney function was well preserved despite that the glomeruli were coated with PEG-phospholipid. Given the established link between the morphological changes after ischemic damage and kidney function as a tool to distinguish between viable and non-viable kidneys *ex-vivo,* our short-term observation study demonstrate the efficacy of the PEG-phospholipid coating to attenuate I/R attack without any adverse effects on the kidney function.

The effect of the PEG-phospholipid is limited to the cell surface, since the binding is mediated by a hydrophobic interaction. From the monitoring of the fluorescence of the PEG-phospholipid bound in the kidney, both in the short- and long-term models, it was shown that there was a decrease in the intensity during the observation period, indicating that PEG-phospholipid detached from the endothelium. Fluorescence seemed to migrate from the glomeruli to the tubular system finally reaching the base of the tubular cells at 6 hours post transplantation. The fluorescence were found in the urine during the observation period, which supports that the PEG-phospholipid molecules were metabolized by the kidney. In the long-term study significant fluorescence was detected up to 12-24 hours after reperfusion and was completely lost after 48 hours. It may be that initially there is an over-saturation of PEG-phospholipid, which is easily washed away and that the actual protection of the endothelial cells is exercised by molecules giving only a faint fluorescence. This is corroborated by relatively low PEG-phospholipid fluorescence in the short-term study after 6 hours post transplantation combined with a protective effect on innate immune attack as indicated by significant differences in TAT, C3a, and sC5b-9 levels in the blood and in the C3b deposition in the kidney graft. This is also supported by that the efficacy remained in the long-term study up to 72 hours despite of a lower density of PEG-phospholipid inserted into the endothelial surface was observed. Therefore, the long-term stability of the PEG-phospholipids is sufficient.

This short-term study provides proof of concept of the efficacy of the solitaire PEG-phospholipid coating to attenuate I/R injury events by directly suppressing thromboinflammation. The PEG-phospholipid was developed to act as a link and a spacer for regulators of innate immunity bound to cells. Functionalized PEG-phospholipid molecules are very efficient in attenuating various kinds of innate immune and thrombotic reactions, such as complement activation, contact system activation and activation of platelets. For instance, a peptide with affinity for Factor H is inhibiting complement activation and an apyrase is able to reduce thrombotic reactions associated with platelet activation. Combined these two regulators can totally inhibit thromboinflammation triggered in a xenogeneic system when porcine aortic endothelial cells come in contact with human blood in a whole-blood *in vitro* system. Considering the immense effect by the PEG-phospholipid alone, the addition of regulators to the PEG-phospholipid will further improve the construct with regard to inhibiting I/R injury in allogeneic kidney transplantation.

Activation of the contact system, which is a part of innate immunity, might cause serious intraoperative complications, such as cardiovascular derangements and hemodynamic instability post-reperfusion. This phenomenon, which previously has been described as post reperfusion syndrome (PRS), was first observed in liver transplant recipients who experienced severe hemodynamic instability after reperfusion of the liver graft. However, this phenomenon is also observed in kidney and pancreas transplant recipients, who often suffers from instant hemodynamic instability after reperfusion. During PRS, the plasma protein factor XII, pre-kallikrein and high molecular weight kininogen are activated resulting in initiation of coagulation cascade and production of bradykinin, which is a very potent vasodilator causing hemodynamic instability. Consequently, the rapid decrease of systemic arterial pressure results in impaired graft perfusion and prolongation of the warm ischemia of the graft. These patients can suffer from delayed graft function, increased risk for rejection and premature graft loss. In healthy individuals, an increased heart rate can partly compensate for the drop in blood pressure.

In our long-term survival pig studies it was shown that the animals, which were transplanted with PEG-phospholipid treated kidneys, had minimal or no changes of the heart rate and arterial blood pressure after reperfusion. Thus, the PEG-phospholipid product also prevented activation of the contact system and ultimately PRS.

The difference between the different long-term experiments was that the graft in the former experiments was stored in a refrigerator +4°C for 24 h without being submerged into perfusion liquid and that the PEG-phospholipid was administered by perfusion into the vascular system 40 min before transplantation of the kidney. In the latter experiments, the graft was immediately perfused with PEG-phospholipid after procurement and thereafter submerged into PEG-phospholipid dissolved in perfusion liquid for 24 hr at +4°C until transplantation and reperfusion. Already in the first study, there was significantly lower cascade system activation (Figs. 8A to 8C) and cytokine expression (Figs. 9A to 9L) in the PEG-phospholipid-treated animals. However, in the second study, the cascade system activation (Figs. 17A to 17D) and cytokine expression (Figs. 18A to 18G) were much lower as compared to the first study and in addition, PEG-phospholipid treatment resulted in a significant reduction of the cascade system activation (Figs. 17A to 17D) and cytokine expression (Figs. 18A to 18G). Hence, the present experiment clearly demonstrated that the combination of perfusion liquid and PEG-phospholipid was the superior combination. In this experiment it was also shown that cytokine expression was clearly lower directly after reperfusion of the graft of the PEG-phospholipid-treated grafts (Figs. 18A to 18G), indicating that the anti-inflammatory effect was already induced during the cold ischemia period before reperfusion, possibly due to an inhibitory effect on CL-11 during ischemia mention above.

### EXPERIMENT 2

The aim of the experiment is a safety assessment of PEG conjugated with phospholipid (1,2-di-palmitoyl-sn-glycerol-3-phosphatidylethanolamine, DPPE) in rats. A single dose of the compound was administered intravenously and followed by a recovery period of 14 days. In the clinical setting, 2 g of the PEG (MW: 5.0 kDa)/phospholipid compound will be diluted in 1000 mL perfusion solution, which is routinely administered to organ transplants *ex vivo,* before insertion in the recipient. The rats in the low dose group were given as the same concentration, and those in the high dose group 10 times that concentration, respectively.

### Study design

Thirty Sprague Dawley rats of both sexes were purchased from Charles River and transported to Uppsala. The experiment ran for a total of four weeks, starting with an acclimatization period of two weeks, after which the animals were injected in a randomized order either with NaCl, low dose or high dose PEG-phospholipid (day 1). The injections were made in the lateral tail vein. The rats were then repeatedly observed in their cages for 6 hours using an Irwin observation test, which includes an extended clinical examination of neurological and motor functions for signs of acute toxicity. The observers were blinded to the treatment. Clinical examinations five days a week were thereafter continued for two weeks. At the end of the study, blood was collected by cardiac puncture under terminal anesthesia. After euthanasia all animals were immediately subjected to necropsy (day 15).
Group 1: 10 control animals (5 females, 5 males) received NaCl, 1 mL
Group 2: 10 rats (5 females, 5 males) received 2 mg/mL PEG-phospholipid, 1 mL
Group 3: 10 rats (5 females, 5 males) received 20 mg/mL PEG-phospholipid, 1 mL

### Clinical observations

Throughout the study all animals were examined and handled 5 days a week. They were taken out of the cage and put on a piece of synthetic fleece one or two at a time. Three times a week all rats were let into a large play area, 5 rats at a time, for climbing, bathing and exploring. Positive reinforcement was used in form of cornflakes immediately after handling. They underwent a thorough clinical examination at arrival to the animal facility and prior to injection of the substance. Immediately after the injection, the rats were observed for signs of toxicity during six hours at set time-intervals. They were thereafter examined regularly by an experienced veterinarian. No clinical signs of disease or toxicity were observed in any animal during the acclimatization and experimental period.

### Body weight

Body weights of all animals were recorded prior to the intravenous injection and thereafter twice a week during the remainder of the study. The animals' weight gain were of similar magnitude in the three groups.

### Ophthalmological examination

All animals underwent an ophthalmologic examination with biomicroscopy (KOWA-SL17) and ophthalmoscopy (Heine Omega 500) one week prior to, and one week after injecting the PEG-phospholipid. The rats received Mydriacyl^{®} (tropicamide) eye drops, 0.5%, 5 mg/mL before the examination. They were held with gentle manual restraint throughout the procedure.

No signs of toxicity were observed by the ophthalmological examination. Before administration of the substance, one female rat had a corneal opacity in the right eye and two female rats had nuclear opacities of the lens, one in both eyes and the other only in the right eye. No ophthalmological changes were seen after administration of the substance, except for a mild epithelial irritation in one of the females. Two male rats had corneal degeneration, one bilateral and the other one only of the right cornea prior to injection of the substance. One male had nuclear defects of the lens and another one cataract. Intravitreal hemorrhage in the left eye could be seen in one male rat. After administration of the substance no changes could be seen in the male rats and the hemorrhage was resorbed.

### Clinical pathology

At termination of the study blood was taken from all animals by cardiac puncture, one ml of blood was divided into EDTA- and Li-heparin tubes. After centrifugation at 3000 rpm for 10 min, plasma was collected and stored in -20°C until analysis of clinical biochemistry. The EDTA blood was immediately analyzed for hematology with Advia 2120 Siemens health care instrument and plasma with Architect c4000, Abbott Laboratories, according to the user manuals and the SOPs at the Department of Clinical Pathology, SLU, Uppsala. The analyses are validated for rodents. The following hematological examinations were made: B-thrombocytes (B-TPK), B-erythrocytes (B-EPK), B-hemoglobin (B-Hb), B-mean corpuscular volume (B-MCV), B-mean corpuscular hemoglobin concentration (B-MCHC), eryreticulocytes, red cell distribution width (RDW), mean corpuscular hemoglobin (MCH), mean platelet volume (MPV), ery-morphology, B-leukocyte (B-LPK), B-neutrophils, B-eosinophils, B-basophils, B-lymphocytes, B-monocytes, and for clinical biochemistry the following determinations were performed: sodium, potassium, glucose, total cholesterol, urea, creatinine, total protein and albumin, alanine aminotransferase (ALAT), aspartate aminotransferase (ASAT) and bile acids. When possible, the following urine analyses were performed: pH, protein, glucose and blood.

The results from all blood and plasma analyses were within the reference range for Sprague Dawley rats aged 8-16 weeks. In five urine samples blood was detected.

### Pathology

All animals were euthanized for scheduled necropsy on day 15 by exsanguination under isoflurane and oxygen anesthesia. Body weights were recorded after euthanasia and gross pathology performed. No macroscopic abnormalities were found in any of the animals.

### Conclusion

The clinical examinations, including ophthalmological investigations, did not reveal any sign of toxicity in neither the low dose, high dose nor control groups of animals. The hematological and biochemical analyses were all within reference ranges and no macroscopic abnormalities were found at necropsy in any animal. It can be concluded that intravenously administered PEG-phospholipid is well tolerated in rats at a concentration up to 20 mg/mL.

### EXPERIMENT 3

Gallium-68 (⁶⁸Ga) is a positron emitter with a half-life of 68 min, which is commonly used as a diagnostic radionuclide in positron-emission tomography (PET)/computed tomography (CT). A radiolabelled PEG-phospholipid molecule, ⁶⁸Ga-NO2A-PEG-phospholipid was produced in a two-step synthesis.

### Preparation of buffers and stock solutions.

*Hydrochloric acid (HCl) 0.1 M for ⁶⁸Ga-elution.* Concentrated HCl (9.00 mL,d=1.17 g×mL⁻¹, 11.23 M, 32-35% Ultrapure NORMATOM for trace metal analysis) was added to water (1000 mL, Thermo Fisher, Ultra Trace Analytical Grade) and mixed and stored at 4°C in the dark directly in the water flask. An aliquot was kept in RT in a plastic flask (Nalgene High Density Polyethylene low metal resin, Thermo Fischer) prior generator elution.

*Metal free water.* Deionized water was obtained in a glass flask from a water purifier (MillieQ). An aliquot was transferred to a plastic container and stored over Chelex-100 to scavenge metal ions.

*NO2A-azide 1.0 M solution in DMSO and water.* 1,4,7-Triazacyclononane-1,4-bis(acetic acid)-7-(3-azidopropylacetamide) trihydrochloride (NO2A-azide, Macrocyclics, formula weight 494.8 g/mol) (1.0 mg, 2.0 µmol) was added to an Eppendorf tube (2 mL). The solid was first dissolved in dimethyl sulfoxide (DMSO, 50 µL) and the diluted very slowly dropwise with metal free water (1950 µL) until a homogenous 1.0 M-solution was obtained.

*Sodium acetate 1.0 M buffer pH 3.5.* Metal free water was used in all steps in the preparation of the buffer. Sodium acetate (8.203 g, 0.100 mol, 99.995% trace metal basis, TraceSELECT) was dissolved in water (100 mL) in a plastic bottle (Nalgene, HDPE low metal resin) and the pH was adjusted to 4.5, 4.0 or 3.5 by addition of concentrated acetic acid (≥99%, TraceSELECT for trace analysis) using a plastic pipette. The final volume was more than 100 mL because of the addition of the salt and acid hence the molarity of the final solution was less than 1.0 M. Approximately 5 mL aliquots of the buffer were sequentially withdrawn and pH was measured with a calibrated pH meter (Mettler Toledo). The aliquots were discarded after pH measurement to avoid any metal contamination from the pH probe. The final buffer was stored over Chelex 100 and filtered through a 0.5 µm syringe filter before further use.

*Sodium hydroxide 1.0 M solution.* Sodium hydroxide solution (100 µL, 10 M, biological extra grade, Sigma Aldrich) was diluted with metal free water (900 µL) in an Eppendorf tube.

### Elution of 68Ga-generator

⁶⁸Ga was eluted from the ⁶⁸Ga/⁶⁸Ge-generator (50 mCi generator, Obninsk, Russia) with 0.1 M HCl (5.0 mL, approx. 0.8 mL/min) using a 10 mL plastic syringe and a motorized syringe driver (Univentor 864, Malta). The resulting solution was fractionated in Eppendorf tubes to obtain a small volume and high radioactivity concentration.
Fraction 1: time 0 - 45 s, 600 µl, 0 MBq - waste
Fraction 2: time 45 -1 min, 15 s, 400 µl, 212 MBq - kept for labelling
Fraction 3: time 1 min, 15 s - 6 min, 15 s, 4000 µl, 281 MBq - waste

### HPLC-method

LaPrep Sigma HPLC system (Hitachi), equipped with a manual injector (Rehodyne 7225i, 20 µL loop size), UV-detector (4D LWL, Knauer) with an external analytical UV-flow cell (2 µL) connected with optical cables, and a radioactivity detector (Flow Count, Bioscan) quipped with an extended range module FC 6106 (Eckert and Ziegler) and a PMT/Nal well detector FC-3300 (Eckert and Ziegler) which was attached in series after the UV-detector. A stainless steel loop was preferred over a plastic loop inside the radio detector due to stickiness of the radioactive materials analyzed. The HPLC apparatus was software controlled using OpenLAB (Agilent) and the results were analysed using the same software.

Ion chamber (VDC 202, Veenstra Instruments, calibration factor for 68Ga used was 751).

Method A: Reversed phase, C18 column YMC-Pack ODS-AL, AL12S05-1046WT, AL-301, (YMC Europe GmbH) 100×4.6 mm (I.D), 5 µm, 120A. Solvent A: Water and 0.1 % TFA, solvent B: Acetonitrile and 0.1% TFA. Solvent gradient 5-70 % B over 5 min, then 70% B for 3 min. Flowrate 1.0 ml·min⁻¹. Method B: Size exclusion chromatography, column: Superdex^{™} Peptide 10/300 GL, GE Healthcare. Isocratic eluent 30% B. Flow rate 0.80 mL·min⁻¹.

### Synthesis of [68Ga]NO2A-azide

Sodium hydroxide 1.0 M (40 µL, 40 µmol) and sodium acetate buffer 0.10 M (25 µL, pH 3.5) were mixed in an Eppendorf tube (2 mL), and added to ⁶⁸Ga (179 MBq) in 0.1 M HCl (400 µL). After that NO2A-azide solution (10 µL, 1.0 M in DMSO/pure water mixture (1/39) by volume) was added (total reaction volume 250 µL) and the vial was vortexed, centrifuged and heated to 80°C in a heating block for 15 min to form the ⁶⁸Ga-labelled NO2A-azide, see Fig. 14 The crude reaction mixture was analyzed using radio-HPLC method A, wavelength λ = 215 nm, t_{R}: no signal (UV), 3.7 min (radio) and was used for subsequent reactions without further purification. Radio-HPLC, Method B was used to have a reference of unreacted 1 in the click reaction, t_{R}: no signal (UV) t_{R}: 25.7 min (radio).

### Synthesis of DBCO-PEG-Lipid

Mal-PEG-lipid (20 mg) was reacted with cysteine (2 mg) in PBS (1 mL) for 1 hr at RT, and purified by spin column (Protein Desalting Spin Column, Thermo Fisher Scientific) to obtain Cys-PEG-lipid after freeze-drying. Then, Cys-PEG-lipid (10 mg in PBS) was reacted with DBCO-C6-NHS ester (6 mg, Click Chemistry Tools LLC) in DMSO (1 mL) for 1 hr at RT. After adding by 10 mL of pure water, the precipitation was removed by filtration (pore size: 0.2 µm, Millex^{®}-GV, Merck Millipore Ltd.), followed by drying in vacuum to obtain DBCO-PEG-lipid (5 mg). The labelling efficiency DBCO was 80 %, which was calculated from the UV absorption of DBCO.

### Synthesis of ⁶⁸Ga-NO2A-PEG-Lipid

DBCO-PEG-Lipid, (2.0 mg, 333 nmol lipid, 75% DBCO functionalization, 250 nmol DBCO) and PBS (50 µL) was added to an Eppendorf tube (2 mL). A centrifuge was used to move all material to the bottom of the vial. Subsequently, 450 µL from previously prepared [⁶⁸Ga]NO2A-azide solution (116 MBq, 9.0 nmol) was added. The final DBCO/azide ratio in the reaction mixture was 1/28 by molar ratio. The reaction was left in RT for 10 min and then heated to 60°C in a heating block for 10 min.

450 µL of the reaction mixture was transferred to a NAP-5 column (Sephadex^{®} G-25 DNA Grade column pre-equilibrated with 5 mL PBS, cutoff 5 kDa, GE Healthcare), which was eluted with PBS in 200 µL fractions. The desired product ⁶⁸Ga-NO2A-PEG-Lipid, see Fig. 15, was eluted in fractions 2-4 (35.0 MBq, 32% decay corrected radiochemical yield in the click reaction based on the amount of [⁶⁸Ga]NO2A-azide added, 7% non-decay corrected radiochemical yield based on the total amount eluted ⁶⁸Ga) and was analysed on radio-HPLC equipped with a size exclusion chromatography column (Superdex^{™} Peptide 10/300 GL, GE), wavelength λ=254 nm, t_{R}: 21.5 min, >92% radiochemical purity. The major impurity was unreacted [⁶⁸Ga]NO2A-azide (5%) t_{R}: 25.7 min.

### EXPERIMENT 4

PET/ Magnetic Resonance Imaging (MRI) examinations were performed on the basis of the results from *in vitro* studies in order to follow the distribution of the PEG-phospholipid construct in pig kidneys and to estimate the amount of PEG-phospholipid that is distributed in the kidneys.

⁶⁸Ga-NO2A-PEG-lipid (in PBS) was mixed with non-labelled MeO-PEG-lipid (in HTK solution) just before the use (⁶⁸Ga-NO2A-PEG-lipid / MeO-PEG-lipid = 1/4 by molar ratio, [PEG-lipid] = 2.0 mg/mL).

Blood was washed out from the pig kidney with cold PBS, and then, the kidney was flushed with HTK solution at 4°C and placed in a plastic bag filled with HTK solution overnight. A solution of ⁶⁸Ga-NO2A-PEG-Lipid (2 mg/mL, in HTK, 20 mL, at least 10 MBq) was carefully injected through the artery of the pig kidney by a syringe, and incubated for 30 min on ice. After the kidney was washed with cold HTK solution (50 mL), the treated kidney was placed on the stage of PET/MRI apparatus (nanoScan^{®}, Mediso Medical Imaging Systems, Budapest, Hungary). The stage was for a large rat of a small animal PET/3T MRI preclinical scanner. MR scout scan (GRE Multi-FOV) were used to set the bed position for whole kidney scan in PET. Static PET examination, multiple bed scans, was performed for 40 minutes. MRI measurements were performed at 3 T using a whole body of pig kidney transmit-receiver coil. Gradient Echo (GRE MultiFOV) sequence was acquired with following parameters: Number of excitations (NEX): 2; Repetition time (TR): 396 ms; Echo time (TE): 4.15 ms; Flip: 45; Slices: 180; Slice thickness: 1 mm; Field of view (FOV): 64×64 mm²; Spatial resolution: 0.5×0.5 mm²; Acquisition time: 14 min. The list mode PET data was reconstructed using Tera tomo 3D algorithm (4 iterations, voxel size: 0.40 mm, matrix: 212×212×582).

PET and MRI images were analyzed using PMOD (version 3.510; PMOD Technologies Ltd.). Volumes of interest were drawn manually on fused PET-MR images, to include the whole kidney and around medulla region. Tracer distribution in rest of the kidney was estimated by subtracting values from whole kidney and around medulla region.

Uptake in each tissue were expressed in MBq, decay corrected to the time of injection. The amount of radioactivity injected and the effluents from the vein, from all washing steps were measured in a dose calibrator (VDC-405, Veenstra Instrumenten, the Netherlands) and decay corrected to the time of injection. All measurements were performed at RT. After the first measurement by PET/MRI and gamma counter, the kidney was washed again with cold HTK solution (50 mL), and then, the kidney was placed on the stage of PET/MRI apparatus for the second measurement.

### Results

A pig kidney was treated with ⁶⁸Ga-NO2A-PEG-lipid in the same way as kidney transplantation to pig recipients and was then subjected to PET/MRI imaging. After treatment of the kidney with ⁶⁸Ga-NO2A-PEG-lipid by injection and washing out through the artery, imaging was performed to evaluate the distribution of ⁶⁸Ga-NO2A-PEG-lipid. The signal of ⁶⁸Ga-NO2A-PEG-lipid was distributed through the whole kidney, and also a higher signal was particularly detected at the area of the medulla (81 %) compared to the cortex area (19 %). When the same kidney was re-imaged at 90 min after further washing with HTK solution, the imaging result was almost the same. The signal of ⁶⁸Ga-NO2A-PEG-lipid was still distributed through the whole kidney, and a higher signal was detected at the medulla area (93 %) compared to the cortex area (7 %). These results showed that all tissues and vessels in the kidney were coated with ⁶⁸Ga-NO2A-PEG-lipid by injection, and the tissues and vessels in the medulla area were well modified with ⁶⁸Ga-NO2A-PEG-lipid.

### REFERENCES

[1] Miura et al., Encapsulation of islets with ultra-thin polyion complex membrane through polyethylene glycol)-phospholipids anchored to cell membrane. Biomaterials, 2006, 27:5828-5835.
[2] Teramura et al., Behavior of Synthetic Polymers Immobilized on Cell Membrane. Biomaterials, 2008, 29:1345-1355.
[3] Teramura and Iwata, Improvement of graft survival by surface modification with polyethylene glycol)-lipid and urokinase in intraportal islet transplantation. Transplantation. 2011, 91(3):271-278.
[4] Nilsson et al., Autoregulation of thromboinflammation on biomaterial surfaces by a multicomponent therapeutic coating. Biomaterials. 2013, 34(4):985-994.
[5] Engberg et al., Inhibition of complement activation on a model biomaterial surface by streptococcal M protein-derived peptides. Biomaterials. 2009, 30(13):2653-2659.
[6] Teramura and Iwata, Surface Modification of Islets With PEG-Lipid for Improvement of Graft Survival in Intraportal Transplantation. Transplantation. 2009, 88(5):624-630.
[7] Asif et al., Heparinization of cell surfaces with short peptide-conjugated PEG-lipid regulates thromobinflammation in transplantation of human MSCs and hepatocytes. Acta Biomateriala. 2016, 35:194-205.

## Claims

1. An *ex vivo* method of treating an organ or a part of the organ, the method comprising:
*ex vivo* infusing a solution comprising poly(ethylene glycol)-phospholipid (PEG-phospholipid) molecules into a vascular system of the organ or the part of the organ; and
*ex vivo* incubating the solution comprising PEG-phospholipid molecules in the vascular system to enable coating of at least a portion of the endothelial lining of the vascular system with the PEG-phospholipid molecules while keeping the organ or the part of the organ submerged in an organ preservation solution comprising PEG-phospholipid molecules.

2. The method according to claim 1, further comprising:
*ex vivo* infusing an organ preservation solution into the vascular system to flush away non-bound PEG-phospholipid molecules from the vascular system.

3. The method according to claim 1 or 2, further comprising:
*ex vivo* infusing an organ preservation solution into the vascular system prior to *ex vivo* infusing the solution comprising PEG-phospholipid molecules into the vascular system.

4. The method according to any of the claims 1 to 3, wherein *ex vivo* infusing the solution comprises:
*ex vivo* clamping one of an artery and a vein of the vascular system;
*ex vivo* infusing the solution comprising PEG-phospholipid molecules into the other of the artery and the vein; and
*ex vivo* clamping the other of the artery and the vein.

5. The method according to any of the claims 1 to 4, wherein *ex vivo* infusing the solution comprises *ex vivo* infusing a solution comprising from 0.25 mg/mL up to 25 mg/mL, preferably from 0.25 mg/mL up to 10 mg/mL, and more preferably from 0.25 mg/mL up to 5 mg/mL, such as 2 mg/mL, PEG-phospholipid molecules into the vascular system.

6. The method according to any of the claims 1 to 5, wherein *ex vivo* infusing the solution comprises *ex vivo* infusing from 5 mL up to 250 mL, preferably from 5 mL up to 100 mL, and more preferably from 5 mL up to 50 mL, of the solution comprising PEG-phospholipid molecules into the vascular system.

7. The method according to any of the claims 1 to 6, wherein ex *vivo* incubating comprises ex *vivo* incubating the solution comprising PEG-phospholipid molecules in the vascular system while keeping the organ or the part of the organ submerged in the organ preservation solution comprising PEG-phospholipid molecules and in a temperature above 0°C but below 8°C, preferably above 0°C but below 6°C, and more preferably above 0°C but equal to or below 4°C.

8. The method according to any of the claims 1 to 7, wherein the organ preservation solution is selected from the group consisting of a histidine-tryptophan-ketoglutarate (HTK) solution, a citrate solution, a University of Wisconsin (UW) solution, a Collins solution, a Celsior solution, a Kyoto University solution and an Institut Georges Lopez-1 (IGL-1) solution.

9. The method according to any of the claims 1 to 8, wherein ex *vivo* incubating comprises ex *vivo* incubating the solution comprising PEG-phospholipid molecules in the vascular system for a period of time from 10 minutes up to 48 hours, preferably from 20 minutes up to 36 hours, and more preferably from 30 minutes up to 24 hour.

10. The method according to any of the claims 1 to 9, wherein
the PEG-phospholipid molecules are selected from the group consisting of unfunctionalized PEG-phospholipid molecules consisting of PEG-phospholipids, functionalized PEG-phospholipid molecules comprising a respective functionalized molecule attached to PEG of the PEG-phospholipid molecules, and a mixture thereof; and
the functionalized molecule is selected from the group consisting of a heparin-binding peptide, N-terminal 4 to 6 short consensus repeats (SCRs) of complement receptor 1 (CR1), CD46 complement regulatory protein (CD46), complement decay-accelerating factor (DAF), a Factor H binding molecule, an adenosine diphosphate (ADP) degrading enzyme, and a mixture thereof, preferably selected from the group consisting of peptide 5C6, apyrase, cluster of differentiation 39 (CD39), C4b-binding protein (C4BP), and a mixture thereof.

11. The method according to any of the claims 1 to 10, wherein the PEG-phospholipid molecules have a formula (I) or a formula (II): 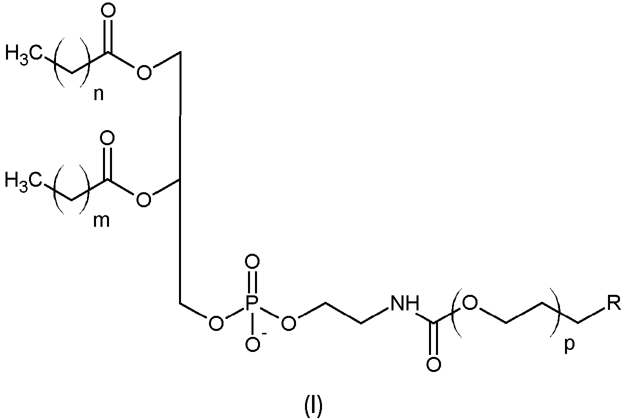 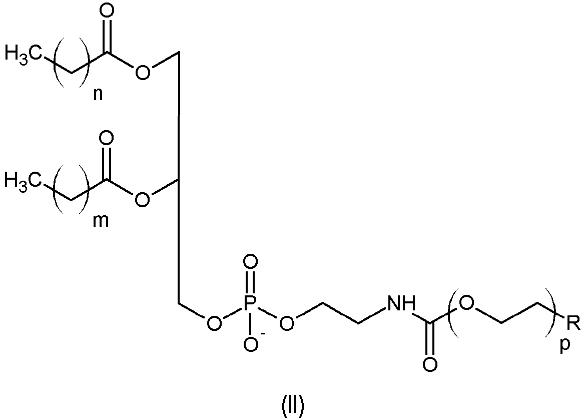 wherein
n, m are integers independently selected within the range of from 10 up to to 16, preferably n, m are independently 10, 12, 14 or 16, and more preferably n=m=14;
p is selected so that the PEG chain has an average molecular weight selected within the range of from 1 000 Da up to 40 000 Da, preferably from 3 000 up to 10 000 Da and more preferably 5 000 Da; and
R is **H,** CH₃, NH₂, a malemide group, a biotin group, a streptavidin group, an avidin group or a functionalized molecule selected from the group consisting of a heparin-binding peptide, N-terminal 4 to 6 short consensus repeats (SCRs) of complement receptor 1 (CR1), CD46 complement regulatory protein (CD46), complement decay-accelerating factor (DAF), a Factor H binding molecule, an adenosine diphosphate (ADP) degrading enzyme, and a mixture thereof, preferably selected from the group consisting of peptide 5C6, apyrase, cluster of differentiation 39 (CD39), C4b-binding protein (C4BP), and a mixture thereof.

12. The method according to any of the claims 1 to 11, wherein the organ is selected from the group consisting of kidney, liver, pancreas, heart, lung, uterus, urinary bladder, thymus and intestine.

13. The method according to claim 12, wherein
the organ is a kidney; and
ex *vivo* incubating comprises ex *vivo* incubating the solution comprising PEG-phospholipid molecules in the vascular system to enable coating of the renal endothelium of the renal vascular system and coating of the tubuli of the renal parenchyma with the PEG-phospholipid molecules while keeping the kidney submerged in an organ preservation solution comprising PEG-phospholipid molecules.

## Patentansprüche

1. Ex-vivo-Verfahren zum Behandeln eines Organs oder eines Teils des Organs, wobei das Verfahren umfasst:
*Ex-vivo*-Infundieren einer Lösung, die Moleküle von Poly(ethylenglykol)phospholipid (PEG-Phospholipid) umfasst, in ein Gefäßsystem des Organs oder des Teils des Organs; und
*Ex-vivo*-Inkubieren der Lösung, die PEG-Phospholipidmoleküle umfasst, in dem Gefäßsystem, um eine Beschichtung zumindest eines Teils der Endothelauskleidung des Gefäßsystems mit den PEG-Phospholipidmolekülen zu ermöglichen, während das Organ oder der Teil des Organs in eine Organkonservierungslösung getaucht gehalten wird, die PEG-Phospholipidmoleküle umfasst.

2. Verfahren nach Anspruch 1, ferner umfassend:
*Ex-vivo*-Infundieren einer Organkonservierungslösung in das Gefäßsystem, um nicht gebundene PEG-Phospholipidmoleküle aus dem Gefäßsystem zu spülen.

3. Verfahren nach Anspruch 1 oder 2, ferner umfassend:
*Ex-vivo*-Infundieren einer Organkonservierungslösung in das Gefäßsystem vor dem *Ex-vivo*-Infundieren der Lösung, die PEG-Phospholipidmoleküle umfasst, in das Gefäßsystem.

4. Verfahren nach einem der Ansprüche 1 bis 3, wobei das *Ex-*vivo-Infundieren der Lösung umfasst:
Ex-vivo-Abklemmen eines einer Arterie und einer Vene des Gefäßsystems;
*Ex-vivo*-Infundieren der Lösung, die PEG-Phospholipidmoleküle umfasst, an das andere der Arterie und der Vene; und
Ex-vivo-Abklemmen des anderen der Arterie und der Vene.

5. Verfahren nach einem der Ansprüche 1 bis 4, wobei das *Ex-vivo*-Infundieren der Lösung das *Ex-vivo*-Infundieren einer Lösung, die 0,25 mg/ml bis zu 25 mg/ml, vorzugsweise 0,25 mg/ml bis zu 10 mg/ml und stärker bevorzugt 0,25 mg/ml bis zu 5 mg/ml, wie z. B. 2 mg/ml, PEG-Phospholipidmoleküle umfasst, in das Gefäßsystem umfasst.

6. Verfahren nach einem der Ansprüche 1 bis 5, wobei das *Ex-vivo*-Infundieren der Lösung das *Ex-vivo*-Infundieren von 5 ml bis zu 250 ml, vorzugsweise 5 ml bis zu 100 ml und stärker bevorzugt 5 ml bis zu 50 ml der Lösung, die PEG-Phospholipidmoleküle umfasst, in das Gefäßsystem umfasst.

7. Verfahren nach einem der Ansprüche 1 bis 6, wobei das *Ex-vivo*-Inkubieren das *Ex-vivo*-Inkubieren der Lösung, die PEG-Phospholipidmoleküle umfasst, in dem Gefäßsystem umfasst, während das Organ oder der Teil des Organs in die Organkonservierungslösung, die PEG-Phospholipidmoleküle umfasst, getaucht und bei einer Temperatur über 0 °C, aber unter 8 °C, vorzugsweise über 0 °C, aber unter 6 °C, und stärker bevorzugt über 0 °C, aber höchstens 4 °C, gehalten wird.

8. Verfahren nach einem der Ansprüche 1 bis 7, wobei die Organkonservierungslösung aus der Gruppe ausgewählt ist, die aus einer Histidin-Tryptophan-Ketoglutarat-Lösung (HTK-Lösung), einer Citratlösung, einer University-of-Wisconsin-Lösung (UW-Lösung), einer Collins-Lösung, einer Celsior-Lösung, einer Kyoto-University-Lösung und einer Institut-Georges-Lopez-1-Lösung (IGL-1-Lösung) besteht.

9. Verfahren nach einem der Ansprüche 1 bis 8, wobei das *Ex-vivo*-Inkubieren das *Ex-vivo*-Inkubieren der Lösung, die PEG-Phospholipidmoleküle umfasst, in dem Gefäßsystem für einen Zeitraum von 10 Minuten bis zu 48 Stunden, vorzugsweise 20 Minuten bis zu 36 Stunden und stärker bevorzugt 30 Minuten bis zu 24 Stunden umfasst.

10. Verfahren nach einem der Ansprüche 1 bis 9, wobei
die PEG-Phospholipidmoleküle aus der Gruppe ausgewählt sind, die aus nicht funktionalisierten PEG-Phospholipidmolekülen, bestehend aus PEG-Phospholipiden, funktionalisierten PEG-Phospholipidmolekülen, umfassend ein jeweiliges funktionalisiertes Molekül, das an PEG der PEG-Phospholipidmoleküle angebunden ist, und einer Mischung davon besteht; und
das funktionalisierte Molekül aus der Gruppe ausgewählt ist, die aus einem Heparinbindungspeptid, N-terminalen 4 bis 6 kurzen Konsensuswiederholungen (SRCs) des Komplementrezeptors 1 (CR1), komplementregulatorischem CD46-Protein (CD46), komplementzerfallbeschleunigendem Faktor (DAF), einem Faktor-H-Bindungsmolekül, einem Adenosindiphosphat (ADP) abbauenden Enzym und einer Mischung davon besteht, vorzugsweise ausgewählt aus der Gruppe, die aus Peptid 5C6, Apyrase, Cluster of Differentiation 39 (CD39), C4b-Bindungsprotein (C4BP) und einer Mischung davon besteht.

11. Verfahren nach einem der Ansprüche 1 bis 10, wobei die PEG-Phospholipidmoleküle eine Formel (I) oder eine Formel (II) aufweisen: 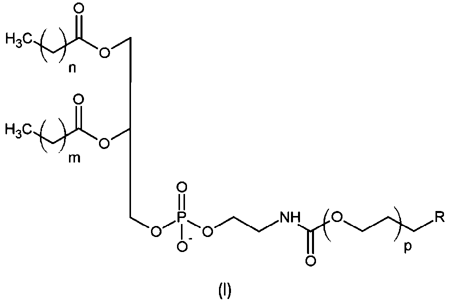 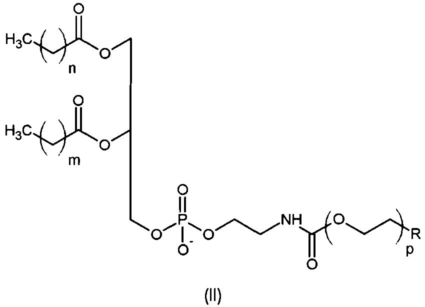 wobei
n, m ganze Zahlen sind, die unabhängig aus dem Bereich von 10 bis zu 16 ausgewählt sind, n, m vorzugsweise unabhängig 10, 12, 14 oder 16 sind und stärker bevorzugt n = m = 14;
p so gewählt ist, dass die PEG-Kette ein durchschnittliches Molekulargewicht aufweist, das aus dem Bereich von 1.000 Da bis zu 40.000 Da, vorzugsweise 3.000 bis zu 10.000 Da und stärker bevorzugt 5.000 Da ausgewählt ist; und
R H, CH₃, NH₂, eine Malemidgruppe, eine Biotingruppe, eine Streptavidingruppe, eine Avidingruppe oder ein funktionalisiertes Molekül ist, das aus der Gruppe ausgewählt ist, die aus einem Heparinbindungspeptid, N-terminalen 4 bis 6 kurzen Konsensuswiederholungen (SRCs) des Komplementrezeptors 1 (CR1), komplementregulatorischem CD46-Protein (CD46), komplementzerfallbeschleunigendem Faktor (DAF), einem Faktor-H-Bindungsmolekül, einem Adenosindiphosphat (ADP) abbauenden Enzym und einer Mischung davon besteht, vorzugsweise ausgewählt aus der Gruppe, die aus Peptid 5C6, Apyrase, Cluster of Differentiation 39 (CD39), C4b-Bindungsprotein (C4BP) und einer Mischung davon besteht.

12. Verfahren nach einem der Ansprüche 1 bis 11, wobei das Organ aus der Gruppe ausgewählt ist, die aus Niere, Leber, Bauchspeicheldrüse, Herz, Lunge, Gebärmutter, Harnblase, Thymus und Gedärm besteht.

13. Verfahren nach Anspruch 12, wobei:
das Organ eine Niere ist; und
das *Ex-vivo*-Inkubieren das *Ex-vivo*-Inkubieren der Lösung, die PEG-Phospholipidmoleküle umfasst, in dem Gefäßsystem umfasst, um eine Beschichtung des Nierenendothels des Nierengefäßsystems und eine Beschichtung der Tubuli des Nierenparenchyms mit den PEG-Phospholipidmolekülen zu ermöglichen, während die Niere in eine Organkonservierungslösung getaucht gehalten wird, die PEG-Phospholipidmoleküle umfasst.

## Revendications

1. Procédé *ex vivo* de traitement d'un organe ou d'une partie de l'organe, le procédé comprenant :
la perfusion *ex vivo* d'une solution comprenant des molécules de poly(éthylène glycol)-phospholipide (PEG-phospholipide) dans un système vasculaire de l'organe ou de la partie de l'organe ; et
l'incubation *ex vivo* de la solution comprenant des molécules de PEG-phospholipide dans le système vasculaire pour permettre le revêtement d'au moins une partie de la muqueuse endothéliale du système vasculaire avec les molécules de PEG-phospholipide tout en maintenant l'organe ou la partie de l'organe immergé dans une solution pour la conservation d'organes comprenant des molécules de PEG-phospholipide.

2. Procédé selon la revendication 1, comprenant en outre :
la perfusion *ex vivo* d'une solution pour la conservation d'organes dans le système vasculaire pour chasser les molécules de PEG-phospholipide non liées du système vasculaire.

3. Procédé selon la revendication 1 ou 2, comprenant en outre :
la perfusion *ex vivo* d'une solution pour la conservation d'organes dans le système vasculaire avant la perfusion *ex vivo* de la solution comprenant des molécules de PEG-phospholipide dans le système vasculaire.

4. Procédé selon l'une quelconque des revendications 1 à 3, dans lequel la perfusion *ex vivo* de la solution comprend :
le clampage *ex vivo* de l'une parmi une artère et une veine du système vasculaire ;
la perfusion *ex vivo* de la solution comprenant des molécules de PEG-phospholipide dans l'autre parmi l'artère et la veine ; et
le clampage *ex vivo* de l'autre parmi l'artère et la veine.

5. Procédé selon l'une quelconque des revendications 1 à 4, dans lequel la perfusion *ex vivo* de la solution comprend la perfusion *ex vivo* d'une solution comprenant de 0,25 mg/ml à 25 mg/ml, de préférence de 0,25 mg/ml à 10 mg/ml, et de manière davantage préférée de 0,25 mg/ml à 5 mg/ml, comme, par exemple, 2 mg/ml, de molécules de PEG-phospholipide dans le système vasculaire.

6. Procédé selon l'une quelconque des revendications 1 à 5, dans lequel la perfusion *ex vivo* de la solution comprend la perfusion *ex vivo* de 5 ml à 250 ml, de préférence de 5 ml à 100 ml, et de manière davantage préférée de 5 ml à 50 ml, de la solution comprenant des molécules de PEG-phospholipide dans le système vasculaire.

7. Procédé selon l'une quelconque des revendications 1 à 6, dans lequel l'incubation *ex vivo* comprend l'incubation *ex vivo* de la solution comprenant des molécules de PEG-phospholipide dans le système vasculaire tout en maintenant l'organe ou la partie de l'organe immergé dans la solution pour la conservation d'organes comprenant des molécules de PEG-phospholipide et à une température supérieure à 0°C mais inférieure à 8°C, de préférence supérieure à 0°C mais inférieure à 6°C, et de manière davantage préférée supérieure à 0°C mais égale ou inférieure à 4°C.

8. Procédé selon l'une quelconque des revendications 1 à 7, dans lequel la solution pour la conservation d'organes est choisie dans le groupe constitué d'une solution d'histidine-tryptophane-cétoglutarate (HTK), une solution de citrate, une solution de l'Université du Wisconsin (UW), une solution de Collins, une solution de Celsior, une solution de l'Université de Kyoto et une solution de l'Institut Georges Lopez-1 (IGL-1).

9. Procédé selon l'une quelconque des revendications 1 à 8, dans lequel l'incubation *ex vivo* comprend l'incubation *ex vivo* de la solution comprenant des molécules de PEG-phospholipide dans le système vasculaire pendant une période allant de 10 minutes à 48 heures, de préférence de 20 minutes à 36 heures, et de manière davantage préférée de 30 minutes à 24 heures.

10. Procédé selon l'une quelconque des revendications 1 à 9, dans lequel
les molécules de PEG-phospholipide sont choisies dans le groupe constitué de molécules de PEG-phospholipide non fonctionnalisées composées de PEG-phospholipides, de molécules de PEG-phospholipide fonctionnalisées comprenant une molécule fonctionnalisée respective fixée au PEG des molécules de PEG-phospholipide, et d'un mélange de celles-ci ; et
la molécule fonctionnalisée est choisie dans le groupe constitué d'un peptide de liaison à l'héparine, de 4 à 6 courtes répétitions consensus N-terminales (SCR) du récepteur du complément 1 (CR1), de la protéine régulatrice du complément CD46 (CD46), du facteur d'accélération de la dégradation du complément (DAF), d'une molécule se liant au Facteur H, d'une enzyme dégradant l'adénosine diphosphate (ADP), et d'un mélange de ceux-ci, est choisie de préférence dans le groupe constitué du peptide 5C6, de l'apyrase, du groupe de différenciation 39 (CD39), de la protéine de liaison au C4b (C4BP), et d'un mélange de ceux-ci.

11. Procédé selon l'une quelconque des revendications 1 à 10, dans lequel les molécules de PEG-phospholipide sont de formule (I) ou de formule (II) : 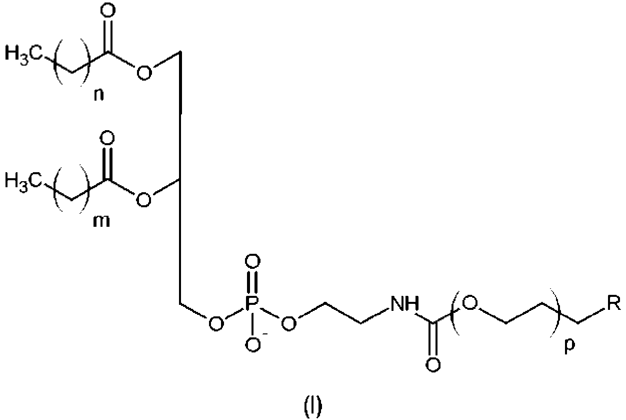 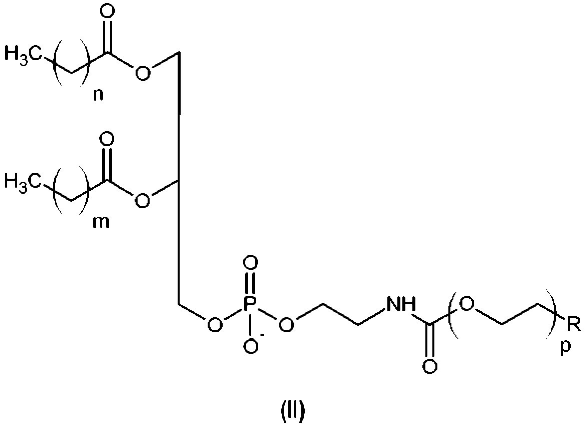 dans laquelle
n, m sont des nombres entiers choisis indépendamment l'un de l'autre dans la plage de 10 à 16, de préférence n, m 15 sont indépendamment l'un de l'autre 10, 12, 14 ou 16, et de manière davantage préférée n = m = 14 ;
p est choisi de sorte que la chaîne de PEG a un poids moléculaire moyen choisi dans la plage allant de 1 000 Da à 40 000 Da, de préférence de 3 000 à 10 000 Da et de manière davantage préférée de 5 000 Da ; et
R correspond à H, CH₃, NH₂, un groupe malémide, un groupe biotine, un groupe streptavidine, un groupe avidine ou une molécule fonctionnalisée choisie dans le groupe constitué d'un peptide de liaison à l'héparine, de 4 à 6 courtes répétitions consensus N-terminales (SCR) du récepteur du complément 1 (CR1), de la protéine régulatrice du complément CD46 (CD46), du facteur d'accélération de la dégradation du complément (DAF), d'une molécule se liant au Facteur H, d'une enzyme dégradant l'adénosine diphosphate (ADP), et d'un mélange de ceux-ci, choisie de préférence dans le groupe constitué du peptide 5C6, de l'apyrase, du groupe de différenciation 39 (CD39), de la protéine de liaison au C4b (C4BP), et d'un mélange de ceux-ci.

12. Procédé selon l'une quelconque des revendications 1 à 11, dans lequel l'organe est choisi dans le groupe constitué du rein, du foie, du pancréas, du cœur, du poumon, de l'utérus, de la vessie, du thymus et de l'intestin.

13. Procédé selon la revendication 12, dans lequel
l'organe est un rein ; et
l'incubation *ex vivo* comprend l'incubation *ex vivo* de la solution comprenant des molécules de PEG-phospholipide dans le système vasculaire pour permettre le revêtement de l'endothélium rénal du système vasculaire rénal et le revêtement des tubuli du parenchyme rénal avec les molécules de PEG-phospholipide tout en maintenant le rein immergé dans une solution pour la conservation d'organes comprenant des molécules de PEG-phospholipide.
